# EUROPEAN PATENT APPLICATION

(11) **EP 4 616 939 A1**
(43) Date of publication of application: **17.09.2025**
(21) Application number: 25163756.7
(22) Date of filing: 14.03.2025
(51) Int. Cl.: B01J 19/00

(54) **NUCLEOTIDES CHIP AND PREPARATION METHOD THEREFOR**

(30) Priority: 15.03.2024 CN 202410307375
(71) Applicant: GeneMind Biosciences Company Limited, Shenzhen, Guangdong 518023 (CN)
(72) Inventor: Gu, Jiaqiang, Shenzhen City, 518023 (CN); Wang, Wenbing, Shenzhen City, 518023 (CN); Lin, Zhifeng, Shenzhen City, 518023 (CN); Zeng, Dezhi, Shenzhen City, 518023 (CN)
(74) Representative: Schiweck Weinzierl Koch Patentanwälte Partnerschaft mbB

(57) **Abstract**

The present application provides a chip, which includes a chip substrate, wherein the chip substrate is provided with regularly arranged depressions on at least one surface; the depression comprises, in sequence, a first geometric structure and a second geometric structure that are interconnected in a direction from a surface of the chip substrate toward a central part of the chip substrate. In the chip provided by the examples of the present application, the regularly arranged depressions are formed on the substrate, and the depression comprises, in sequence, the first geometric structure and the second geometric structure that are interconnected in the direction from the surface of the chip substrate toward the central part of the chip substrate, so that the morphological structure of the depressions in the present application facilitates substance exchange and diffusion between the liquid phase of a reaction reagent and active molecules on the surface of the chip during a biochemical reaction in a detection process (e.g., nucleic acid sequencing), thereby promoting the reaction to occur and thus obtaining better detection signals (including signal intensity and signal-to-noise ratio) and more accurate detection results.

## Description

### TECHNICAL FIELD

The present disclosure relates to the field of biotechnology, and in particular to, a chip and a method for preparing same.

### BACKGROUND

Chips for biomolecule detecting platforms are based on the principle of specific interaction between biomolecules and integrate biochemical analysis processes on the chip surface to achieve high-throughput, fast detection of DNA, RNA, polypeptide, protein, and other biological components. Biochips possess the advantages of high throughput, miniaturization, automation, and the like. They are widely used in the fields of gene sequencing, protein detection, small biomolecule (such as saccharides) detection, and the like, and provide feasible technical schemes for the techniques of nucleic acid sequence analysis, drug screening, and large-scale biological sample detection.

Biomolecule detection sites can be formed on the chip surface through geometric structures with specific shapes. A patterned arrangement of the sites may facilitate the control of the arrangement density of the geometric structures on the chip surface and can thereby achieve the simultaneous detection of various samples, thus improving the detection throughput. Configuring the chip surface as a patterned surface with a regular arrangement of geometric structures is beneficial to improving the arrangement density of the chips and improving the surface utilization rate.

The detection of nucleic acid sequence on chip surface features a broad application range. Studies have shown that the structural design of chips, as a medium and a reactor of sequencing, will influence various parameters and indicators reflecting the sequencing results, thus affecting the accuracy of the sequencing results. How to design a proper chip micro/nano structure to improve the sequencing stability and accuracy upon use of the chip in sequencing, has become a concern of those skilled in the art.

### SUMMARY

Through studies, the inventor has found that in a chip-based method for nucleic acid sequencing, the shape and dimensions of the geometric structure on the surface of the chip, including the height of the geometric structure in the vertical direction, the dimension of the geometric structure in the horizontal direction, the depth of the geometric structure, and the shape of the geometric structure (e.g., whether the geometric structure is narrower at the top and wider at the bottom, wider at the top and narrower at the bottom, or consistent in width from the top to the bottom), may affect the stability and accuracy of the sequencing results. Such an effect is reflected by the sequencing indicators or parameters obtained during the sequencing process, such as: the stability of clonal clusters obtained after the amplification of nucleic acid molecules within the geometric structure, the intensity of signals captured during the sequencing, the quality parameters of sequencing sequences (reads) such as Q30, the splitting rate of multiple tags on samples, the alignment rate of sequencing results with a reference genome, etc.

In view of this, the present disclosure provides a chip and a method for preparing same advantageous to improving the sequencing quality by designing the shape and dimensions of the geometric structure on the chip.

The first aspect of the present disclosure provides a patterned chip, including a chip substrate, where the chip substrate is provided with regularly arranged depressions on at least one surface;
the depression includes, in sequence, a first geometric structure and a second geometric structure that are interconnected in a direction from a surface of the chip substrate toward a central part of the chip substrate; the first geometric structure has a depth c of 0-100 µm in a direction perpendicular to the surface of the chip, a maximum dimension a of 10 nm-100 µm in a direction parallel to the surface of the chip, and a maximum dimension b of 10 nm-100 µm in a horizontal direction on the side away from the surface of the chip; an included angle α between a side wall of the first geometric structure and the horizontal direction on the side away from the surface of the chip is 10-170°;
the second geometric structure has a depth e of 0-100 µm in the direction perpendicular to the surface of the chip and a maximum dimension d of 0-100 µm in the horizontal direction on the side away from the surface of the chip; the depth c and the depth e are not simultaneously 0.

By forming the regularly arranged depression structures on the substrate, the present disclosure can improve the stability of sequencing and the accuracy of sequencing results. Moreover, the regularly arranged depression structures can improve the arrangement density of the depression structures on the chip and thus the detection throughput; the detection sites corresponding to the depression structures can be positioned through the regularly arranged depression structures, thereby facilitating the synchronous detection of multiple types of nucleic acid molecules on the surface of the same chip and improving the detection cost-efficiency.

As one possible embodiment, the depression includes, in sequence, a first geometric structure and a second geometric structure that are interconnected in a direction from a surface of the chip substrate toward a central part of the chip substrate.

As one possible embodiment, the first geometric structure has a depth c of 0-100 µm in a direction perpendicular to the surface, a maximum dimension a of 10 nm-100 µm in a direction parallel to the surface, and a maximum dimension b of 10 nm-100 µm in a horizontal direction on the side away from the surface; an included angle α between a side wall of the first geometric structure and the horizontal direction on the side away from the surface of the chip is 10-170°.

As one possible embodiment, the first geometric structure has a depth c of 0-100 µm in the direction perpendicular to the surface of the chip, a maximum dimension a of 10 nm-100 µm in the direction parallel to the surface of the chip, and a maximum dimension b of 10 nm-100 µm in the horizontal direction on the side away from the surface of the chip; an included angle α between a side wall of the first geometric structure and the horizontal direction on the side away from the surface of the chip is 10-170°.

As one possible embodiment, the second geometric structure has a depth e of 0-100 µm in the direction perpendicular to the surface of the chip and a maximum dimension d of 0-100 µm in the horizontal direction on the side away from the surface of the chip; the depth c and the depth e are not simultaneously 0.

As one possible embodiment, the included angle α satisfies the following relationship: 10° ≤ α < 90°.

As one possible embodiment, the maximum dimension a is greater than the maximum dimension b, and the maximum dimension b is equal to the maximum dimension d; at this point, the shape of the depression is similar to a horn.

As one possible embodiment, when the shape of the depression is similar to a horn, the depth c is 50 nm-50 µm.

As one possible embodiment, the maximum dimension a is greater than the maximum dimension b, the maximum dimension b is greater than the maximum dimension d, and the maximum dimension d is not 0; at this point, the shape of the depression is similar to a truncated cone, i.e., a cone-like shape with a flat bottom at the top.

As one possible embodiment, when the shape of the depression is similar to a truncated cone, the depth c is 50 nm-50 µm.

As one possible embodiment, when the shape of the depression is similar to a truncated cone, the ratio of the depth c to the depth e is 1:1000-1000:1.

As one possible embodiment, the maximum dimension a is greater than the maximum dimension b, and the maximum dimension d is equal to 0; in this case, the shape of the depression is similar to a cone.

As one possible embodiment, when the shape of the depression is similar to a cone, the depth c is 50 nm-50 µm.

As one possible embodiment, when the shape of the depression is similar to a cone, the depth e is 0.

As one possible embodiment, the distance f between adjacent depressions is 10 nm-100 µm. **In** the present application, the distance between adjacent depressions is the center-to-center distance of the depressions.

As one possible embodiment, the surface is provided with at least one array unit having the regularly arranged depressions.

As one possible embodiment, the array unit is one or more of a square array unit, a rectangular array unit, and a honeycomb array unit.

As one possible embodiment, the chip further includes a first cover plate bonded to the surface of the chip substrate provided with the depression, and the first cover plate is made of a material having a light transmittance of 95% or greater.

As one possible embodiment, the surface of the first cover plate facing the chip substrate is a flat surface.

As one possible embodiment, the surface of the first cover plate facing the chip substrate is provided with regularly arranged geometric structures, and the geometric structure of the first cover plate is identical to or different from the depression of the chip substrate.

As one possible embodiment, the chip substrate is provided with regularly arranged geometric structures on both surfaces.

As one possible embodiment, the chip includes a first cover plate and a second cover plate respectively bonded to the two surfaces of the chip substrate, and the first cover plate and the second cover plate are made of a material having a light transmittance of 95% or greater.

As one possible embodiment, two surfaces of the first cover plate and the second cover plate are flat surfaces.

As one possible embodiment, the first cover plate is provided with a geometric structure on one surface, the geometric structure of the first cover plate is arranged opposite to the depression of the chip substrate, and two surfaces of the second cover plate are flat surfaces.

As one possible embodiment, the first cover plate and the second cover plate are both provided with a geometric structure on one surface, and the geometric structures on the first cover plate and the second cover plate are respectively arranged opposite to the depressions on the two surfaces of the chip substrate. As one possible embodiment, the chip includes a polymer layer arranged on the surface provided with the depression.

As one possible embodiment, the polymer layer is formed from a polymer containing a first functional group selected from one or more of sulfydryl, formyl, hydroxyl, carboxyl, amino, alkenyl, alkynyl, and azido.

As one possible embodiment, the polymer layer is bonded to the surface of the chip substrate and/or the cover plate via a coupling molecule.

As one possible embodiment, the coupling molecule has a molecular formula as follows: XₐSi(Y_{b})M_{c}Z, where
X is H, F, Cl, Br, I, C₁₋₆ alkyl, C₁₋₆ haloalkyl, or C₁₋₆ alkoxy;
Y is selected from C₁₋₁₀ alkyl;
M_{c} is selected from C₁₋₅₀ alkylene, -(OCH₂CH₂)_{c}-, and -(CH(OH)CH₂-)_{c};
Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl;
a, b, and c are integers meeting the following conditions: 0 ≤ a ≤ 3, 0 ≤ b ≤ 3, and a + b = 3; 1 ≤ c ≤ 50. As one possible embodiment, the chip further includes a first film layer arranged between the chip substrate and/or the cover plate and the polymer layer, where the first film layer is made of a material different from that of the surface to which it is bonded.

As one possible embodiment, the first film layer is selected from one or more of a metal film, a metal oxide film, and an inorganic oxide film.

As one possible embodiment, the first film layer is made of at least one of Au, Al, and SiO₂.

As one possible embodiment, the first film layer has a thickness of 10-1000 nm.

As one possible embodiment, a glue layer is arranged between the chip substrate and the cover plate, and the glue layer has a channel arranged in a length direction of the chip; the depression is arranged on the surface of the chip substrate in a region where the channel is located and/or the geometric structure is arranged on the surface of the cover plate in a region where the channel is located, and the shapes and/or arrangements of the depressions or the geometric structures in different channels on the same surface are identical or different.

The second aspect of the present disclosure provides a method for preparing a chip, including the following step:
a) forming regularly arranged depressions on at least one surface of a substrate, where the depression includes, in sequence, a first geometric structure and a second geometric structure that are interconnected in a direction from a surface of a chip substrate toward a central part of the chip substrate; the first geometric structure has a depth c of 0-100 µm in a direction perpendicular to the surface of the chip, a maximum dimension a of 10 nm-100 µm in a direction parallel to the surface of the chip, and a maximum dimension b of 10 nm-100 µm in a horizontal direction on the side away from the surface of the chip; an included angle α between a side wall of the first geometric structure and the horizontal direction on the side away from the surface of the chip is 10-170°;
   the second geometric structure has a depth e of 0-100 µm in the direction perpendicular to the surface of the chip and a maximum dimension d of 0-100 µm in the horizontal direction on the side away from the surface of the chip;
   the depth c and the depth e are not simultaneously 0.

As one possible embodiment, the substrate is the chip substrate and/or a cover plate.

As one possible embodiment, the method further includes:
b) forming a polymer layer on the depression.

As one possible embodiment, b) further includes:
bonding a polymer containing a first functional group to the surface provided with the geometric structure; and
polishing the polymer to remove the polymer outside the region where the geometric structure is located, so as to form the polymer layer on the geometric structure.

As one possible embodiment, the polishing is chemical polishing and/or mechanical polishing.

As one possible embodiment, the method further includes: before step a), or after step a) and before step
b), preparing a first film layer on the surface provided with the depression, where the first film layer and the substrate are made of different materials.

As one possible embodiment, the first film layer is selected from one or more of a metal film, a metal oxide film, and an inorganic oxide film.

As one possible embodiment, the first film layer is made of at least one of Au, Al, and SiO₂.

As one possible embodiment, the first film layer has a thickness of 10-1000 nm.

As one possible embodiment, the substrate includes at least the chip substrate, and the method further includes:
bonding the cover plate to the surface of the chip substrate provided with the geometric structure using an adhesive material, and forming a channel in the adhesive material in a length direction of the chip.

As one possible embodiment, the chip is the chip according to the first aspect.

### BRIEF DESCRIPTION OF THE DRAWINGS

FIG. 1 is a schematic cross-sectional view of a chip substrate with cylindrical depression geometric structures provided in an example of the present disclosure;
FIG. 2 is a schematic top view of a chip substrate with cylindrical depression geometric structures provided in an example of the present disclosure;
FIG. 3 is a physical cross-sectional view of a chip substrate with cylindrical depression geometric structures provided in an example of the present disclosure;
FIG. 4 is a physical top view of a chip substrate with cylindrical depression geometric structures provided in an example of the present disclosure;
FIG. 5 is a schematic structural diagram of a depression on the surface of a chip provided in an example of the present application;
FIG. 6 is a schematic structural diagram of the first type of distance between adjacent depressions on the surface of a chip provided in an example of the present application;
FIG. 7 is a schematic structural diagram of the second type of distance between adjacent depressions on the surface of a chip provided in an example of the present application;
FIG. 8 is a schematic cross-sectional view of a chip substrate with the first structure;
FIG. 9 is a physical diagram of a chip with the first structure;
FIG. 10 is a schematic cross-sectional view of a chip substrate with the second structure;
FIG. 11 is a physical diagram of a chip with the second structure;
FIG. 12 is a schematic cross-sectional view of a chip substrate with the third structure;
FIG. 13 is a physical diagram of a chip with the third structure;
FIG. 14 is a schematic cross-sectional view of a chip substrate with the fourth structure;
FIG. 15 is a physical diagram of a chip with the fourth structure;
FIG. 16 is a schematic cross-sectional view of a chip substrate with the fifth structure;
FIG. 17 is a physical diagram of a chip with the fifth structure;
FIG. 18 is a schematic structural diagram of a chip having a chip substrate with geometric structures on one surface provided in an example of the present disclosure;
FIG. 19 is a schematic structural diagram of another chip having a chip substrate with geometric structures on one surface provided in an example of the present disclosure;
FIG. 20 is a schematic structural diagram of a chip having a chip substrate with geometric structures on two surfaces provided in an example of the present disclosure;
FIG. 21 is a schematic structural diagram of another chip having a chip substrate with geometric structures on two surfaces provided in an example of the present disclosure;
FIG. 22 is a schematic structural diagram of yet another chip having a chip substrate with geometric structures on two surfaces provided in an example of the present disclosure;
FIG. 23 is a statistical diagram of fluorescence intensities of nucleic acid amplifications on biochips with different depression shapes provided in Examples 1-5 of the present disclosure;
FIG. 24 is a statistical diagram of Q30, output ratio, splitting rate, and alignment rate of biochips with different depression shapes provided in Examples 1-5 of the present disclosure.

### DETAILED DESCRIPTION

The present disclosure provides a patterned biochip and a method for preparing same, which may be implemented by those skilled in the art with appropriately improved process parameters with reference to the present disclosure. It should be particularly noted that all similar substitutions and modifications will be apparent to those skilled in the art and are considered to be included in the present disclosure. Although the method and use of the present disclosure have been described through preferred examples, it will be apparent that those skilled in the art can make modifications or proper changes and combinations to the method and use disclosed herein to implement and apply the techniques of the present disclosure without departing from the content, spirit, and scope of the present disclosure.

The examples of the present application provide a chip, including a chip substrate, where the chip substrate is configured for carrying a sample under test, such as nucleic acid molecules under test, and providing a reaction site or a detection site for the sample under test. In the examples of the present application, the chip substrate is provided with a depression on at least one surface, and thus, the chip may also be referred to as a patterned chip. In some embodiments, by encapsulating the chip, a fluid channel including a reaction region can be formed, and thus, the chip provided with the depression is also referred to as a flow cell.

In the examples of the present application, the chip substrate has relatively good stability and chemical reaction inertness, so that when a sample under test, such as nucleic acid molecules, is immobilized on the surface of the chip substrate, the material of the chip substrate does not change the physicochemical properties and structure of the sample under test. Taking nucleic acid molecules as an example of the sample under test, the structure of the sample under test includes, but is not limited to, the composition of the nucleic acid molecules, and the spatial conformation, the two-dimensional structure and even the three-dimensional structure of the nucleic acid molecules. In some specific embodiments, the material of the chip substrate includes, but is not limited to: glass; quartz; a silicon-based material, such as silicon dioxide, polycrystalline silicon, monocrystalline silicon, etc.; a polymer, such as hexamethyldisilazane (HMDS), polyethylene (PE), polypropylene (PP), etc.; a metal, such as aluminum, etc. The material may be one of the above materials, or may be a composite substrate material formed by a combination of two or more of the above materials. It should be understood that when the chip substrate consists of a plurality of materials, the composition methods include at least the following two cases. In one embodiment, two or more materials are mixed to form a mixed material, which is used as a starting material to prepare the chip substrate. At this point, the chip substrate is present as one layer of material. Preferably, similar materials may be selected for mixing to prepare the chip substrate in consideration of the processability of the chip substrate and the performance of the resultant product. Illustratively, a mixture containing silicon dioxide and polycrystalline silicon is used to prepare the chip substrate, or a mixture containing polyethylene (PE) and polypropylene (PP) is used to prepare the chip substrate, and so on. In another embodiment, two or more different materials are selected to prepare a composite layer having at least two layer structures as the chip substrate. Each layer structure may be made of one material or, according to the above embodiment, may be made of a composite of two or more different materials. The layer structures may be arranged in a certain structure, such as a laminated structure, etc.

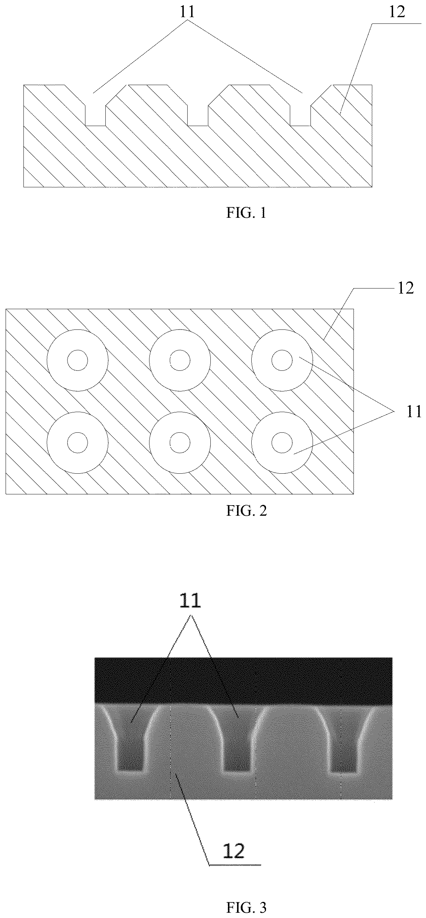

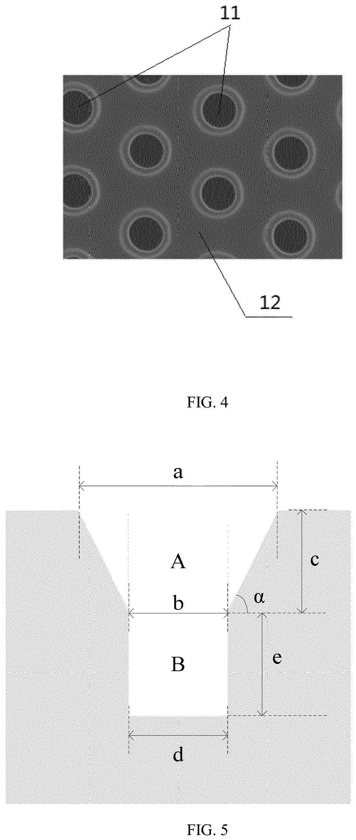

In the examples of the present application, the size of the chip substrate is not particularly limited and can be designed as needed. In some examples, for convenience of processing, regularly arranged geometric structures are formed on a wafer with a large area, and the wafer is then cut to give chip substrates with the required size. The size of the wafer structure is not limited, and may be 6 inches, 8 inches, 12 inches, etc. The chip substrate provided in the examples of the present application is provided with a geometric structure. In the examples of the present application, the shape of the geometric structure on the surface of the chip substrate is a depression formed by an indentation on the surface of the chip substrate, i.e., a depression structure formed on the substrate. Illustratively, taking a depression structure that is wider at the top and narrower at the bottom as an example of the geometric structure, the schematic cross-sectional view and the schematic top view of the chip substrate are respectively shown in FIG. 1 and FIG. 2, where 11 is the depression that is wider at the top and narrower at the bottom, and 12 is the chip substrate; the physical cross-sectional view and the physical top view are respectively shown in FIG. 3 and FIG. 4.

In the examples of the present application, the depression is provided on at least one surface of the chip substrate.

In one embodiment, the depression of the chip substrate is formed on one surface of the chip substrate. At this point, the material of the chip substrate may be selected from the above-listed materials, for example, glass; quartz; a silicon-based material, such as silicon dioxide, polycrystalline silicon, monocrystalline silicon, etc.; a polymer, such as hexamethyldisilazane (HMDS), polyethylene (PE), polypropylene (PP), etc.; a metal, such as aluminum, etc. The chip substrate may be made of one of the above materials, or may be made of a composite material formed by a combination of two or more of the above materials. In some examples, the chip substrate may be provided as a single layer, and the single-layer chip substrate may be made of one material or a mixture of multiple materials; the chip substrate may also be provided as a laminated structure, where each layer forming the laminated structure may be made of one material or a mixture of multiple materials; the depression is provided on the surface layer of the laminated structure and may penetrate the surface, extending down to at least one other laminated layer.

In another embodiment, the depression of the chip substrate is formed on two surfaces of the chip substrate. The resultant chip substrate provides two surfaces for the sample under test to undergo biochemical reactions and/or detections, such as nucleic acid sequencing, thus advantageously improving the throughput of the biochemical reactions and/or detections. In some examples, the chip substrate is a single layer. When the detection of the sample under test requires the use of photoelectric signals, such as when the chip is used for sequencing nucleic acid molecules, the light transmittance of the single-layer chip substrate is less than 70% or the thickness of the single-layer chip substrate is 500 µm or greater, such that when an imaging apparatus collects the optical signals from the two surfaces (especially the geometric structures) of the chip substrate, the interference between the optical signals of the two surfaces is reduced. The single-layer chip substrate may be made of an opaque material, which may be a silicon-based material such as silicon dioxide, polycrystalline silicon, monocrystalline silicon, etc.; or a metal, such as aluminum, etc. The single-layer chip substrate may also be made of various mixed materials, and the light transmittance of the film layer formed by the mixed materials is less than 70% or the thickness of the film layer formed by the mixed materials is 500 µm or greater. For example, a silicon-based material is mixed with other base materials in a proper ratio to give the chip substrate. In another example, the chip substrate is a laminated structure, including at least one layer having a light transmittance of less than 70% or a layer having a thickness of 500 µm or greater, so as to reduce the mutual interference of optical signals generated from the two surfaces (especially the geometric structures) of the chip substrate. Illustratively, the chip substrate is a laminated structure including two layers. In one case, one layer is a layer having a light transmittance of greater than 95%, and the other layer is a layer having a light transmittance of less than 70%; the surfaces of the two layers away from each other are respectively provided with the geometric structure. In another case, both layers are those having a light transmittance of less than 70%; the surfaces of the two layers away from each other are respectively provided with the geometric structure. In still another case, both layers are those having a light transmittance of greater than 95%, and the overall thickness of the two layers is 500 µm or greater; the surfaces of the two layers away from each other are respectively provided with the geometric structure.

In the examples of the present application, the depression has an irregular shape.

Referring to FIGs. 5-17, the examples of the present application provide schematic structural diagrams of the depression on the surface of the chip. In an example of the present application, referring to FIG. 5, the depression includes, in sequence, a first geometric structure A and a second geometric structure B that are interconnected in a direction from a surface of the chip substrate toward a central part of the chip substrate. Here, the direction from the surface of the chip substrate toward the central part of the chip substrate can also be understood as the top-to-bottom direction, i.e., an opening is formed on one surface of the chip substrate, extends toward the other surface of the chip substrate, but does not penetrate through the other surface, thereby forming the depression. As shown in the figure, the first geometric structure A has a depth c in a direction perpendicular to the surface of the chip, a maximum dimension a in a direction parallel to the surface of the chip, and a maximum dimension b in a horizontal direction on the side away from the surface of the chip; an included angle α is formed between a side wall of the first geometric structure and the horizontal direction on the side away from the surface of the chip; the second geometric structure B has a depth e in the direction perpendicular to the surface of the chip and a maximum dimension d in the horizontal direction on the side away from the surface of the chip. In the examples of the present application, the maximum dimension in the direction parallel to the surface of the chip can be understood as the maximum distance among all distances formed between any two points on the edge of the depression in the cross-section formed by the depression on the surface of the chip; similarly, the maximum dimension b of the first geometric structure A in the horizontal direction on the side away from the surface of the chip can be understood as the maximum distance among all distances formed between any two points on the edge of the depression in the shared surface of the first geometric structure A and the second geometric structure B; the maximum dimension d of the second geometric structure B in the horizontal direction on the side away from the surface of the chip can be understood as the maximum distance among all distances formed between any two points on the edge of the bottom surface of the depression.

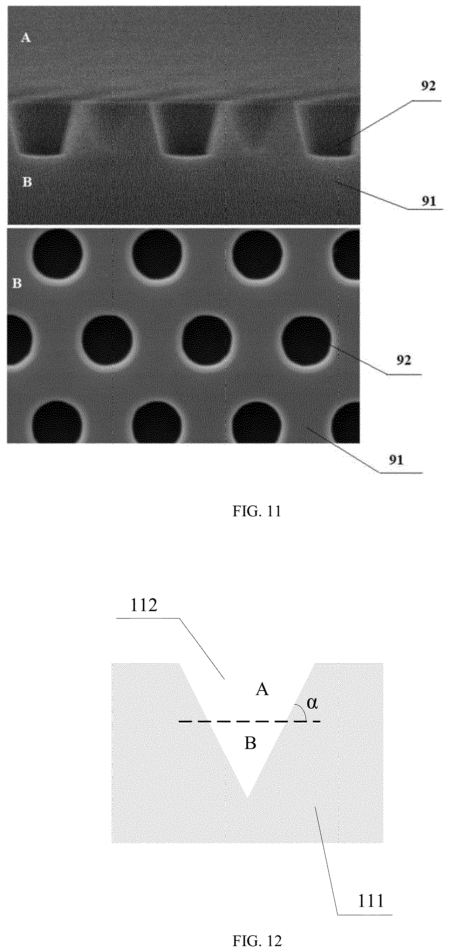

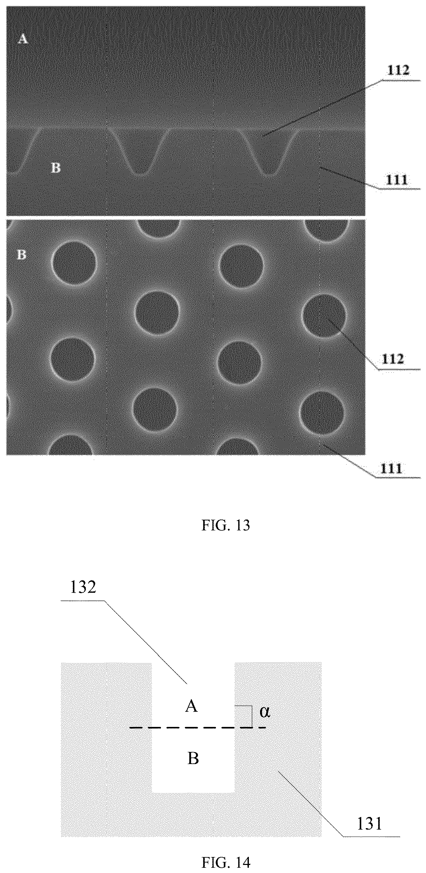

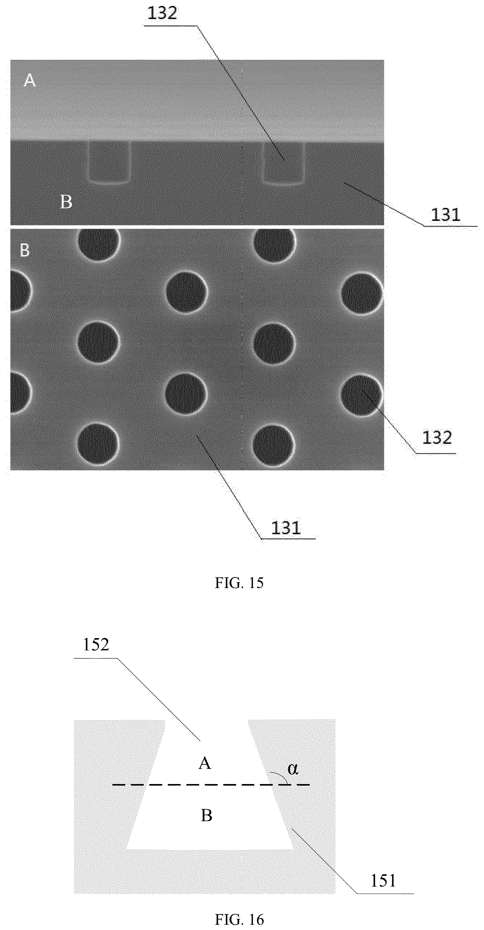

In the examples of the present application, the dimensions of the depression can meet the detection requirements of biomolecules; specifically, the first geometric structure A has a depth c of 0-100 µm in the direction perpendicular to the surface of the chip, a maximum dimension a of 10 mn-100 µm in the direction parallel to the surface of the chip, and a maximum dimension b of 10 nm-100 µm in the horizontal direction on the side away from the surface of the chip; the included angle α between the side wall of the first geometric structure and the horizontal direction on the side away from the surface of the chip is 10-170°. In some examples, the depth c is preferably 10 nm-100 µm, more preferably 50 nm-50 µm, even more preferably 100 nm-10 µm, still more preferably 150 nm-5 µm, and most preferably 200 nm-3 µm. The maximum dimension a is preferably 50 nm-50 µm, more preferably 100 nm-10 µm, even more preferably 150 nm-5 µm, and most preferably 200 nm-3 µm. The maximum dimension b is preferably 50 nm-50 µm, more preferably 100 nm-10 µm, even more preferably 150 nm-5 µm, and most preferably 200 nm-3 µm. In one implementation, as shown in FIGs. 5 and 12, when α < 90 °, the cross-sectional area of the first geometric structure A on the surface of the chip substrate is larger than that inside the chip substrate, and the first geometric structure A is a structure that is wider at the top and narrower at the bottom. In an example of the present application, when the depression has a morphological structure that is wider at the top and narrower at the bottom, it facilitates substance exchange and diffusion between the liquid phase of a reaction reagent and active molecules on the surface of the chip during a biochemical reaction in a detection process (e.g., nucleic acid sequencing), thereby promoting the reaction to occur and thus obtaining better detection signals (including signal intensity and signal-to-noise ratio) and more accurate detection results. Experimental results show that this structure achieves better sequencing results (including Q30 ratio, alignment rate, error rate, splitting rate, output ratio, sequencing signal intensity, etc.). In some examples, as shown in FIG. 5, the included angle α preferably satisfies the following relationship: 10° ≤ α < 90°, more preferably α = 20-80°, even more preferably α = 30-70°, and most preferably α = 40-60°. In another implementation, as shown in FIG. 16, when α > 90 °, the first geometric structure A is a structure that is narrower at the top and wider at the bottom. In yet another implementation, as shown in FIG. 14, when α = 90 °, the first geometric structure A is a cylindrical structure.

In the examples of the present application, the depth e of the second geometric structure in the direction perpendicular to the surface of the chip is 0-100 µm, preferably 10 nm-100 µm, more preferably 50 nm-50 µm, even more preferably 100 nm-10 µm, still more preferably 150 nm-5 µm, and most preferably 200 nm-3 µm. The maximum dimension d is 0-100 µm, preferably 10 nm-100 µm, more preferably 50 nm-50 µm, even more preferably 100 nm-10 µm, still more preferably 150 nm-5 µm, and most preferably 200 nm-3 µm.

In the examples of the present application, the ratio of the maximum dimension b to the maximum dimension d is preferably 1:10-1000:1, more preferably 1:5-500:1, and most preferably 1:1-100:1.

In the examples of the present application, the depth c and the depth e are not simultaneously 0. In some examples, the ratio range of the depth c to the depth e is preferably 1:1000-1000:1, more preferably 1:500-500:1, even more preferably 1:100-100:1, still more preferably 1:50-50:1, further preferably 1:10-10:1, and most preferably 1:5-5:1. In some examples, one of the depth c and the depth e is 0. When the depth c is 0, the depression structure is the second geometric structure B; when the depth e is 0, the depression structure is the first geometric structure A.

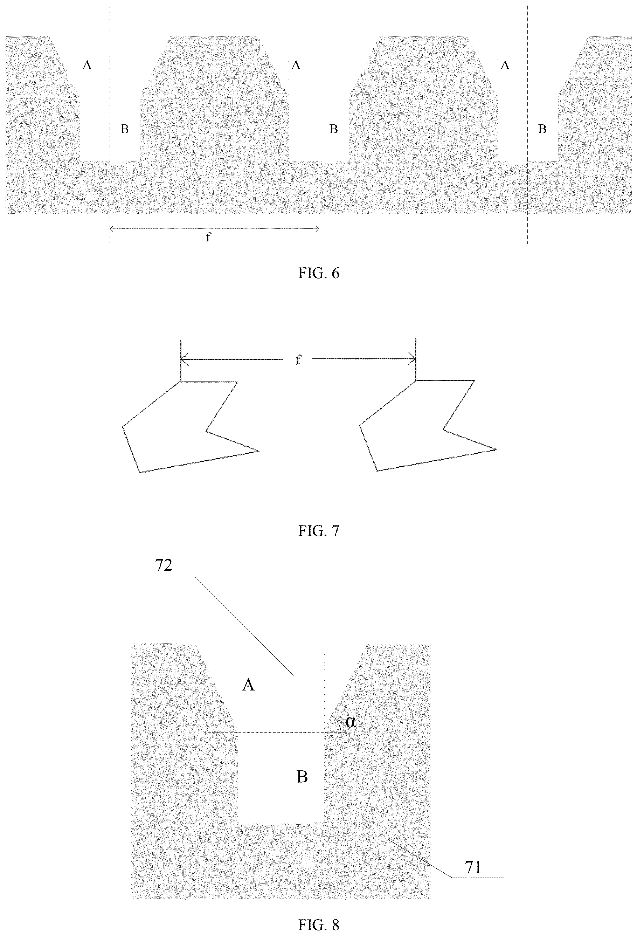

In the examples of the present application, the distance f between adjacent depressions can be divided into two cases according to the shape of the cross-section of the depression. In one implementation, when the cross-section of the depression is a symmetrical pattern, the distance f between adjacent depressions is the distance between the centers of two adjacent depressions, i.e., the distance from the center of one depression to the center of the adjacent depression, as shown in FIG. 6. In another implementation, when the depression has an irregular shape, f is the distance between the same positions in two cross-sections of adjacent depressions formed on the same horizontal plane, as shown in FIG. 7. In some examples, the distance f between adjacent depressions is preferably 10 nm-100 µm, more preferably 50 nm-50 µm, even more preferably 100 nm-10 µm, still more preferably 200 nm-5 µm, and most preferably 300 nm-3 µm.

In the examples of the present application, by adjusting the above parameters, depressions with different structures can be obtained.

In some embodiments of the present application, by adjusting the above parameters, a depression that is wider at the top and narrower at the bottom is obtained on the surface of the chip substrate.

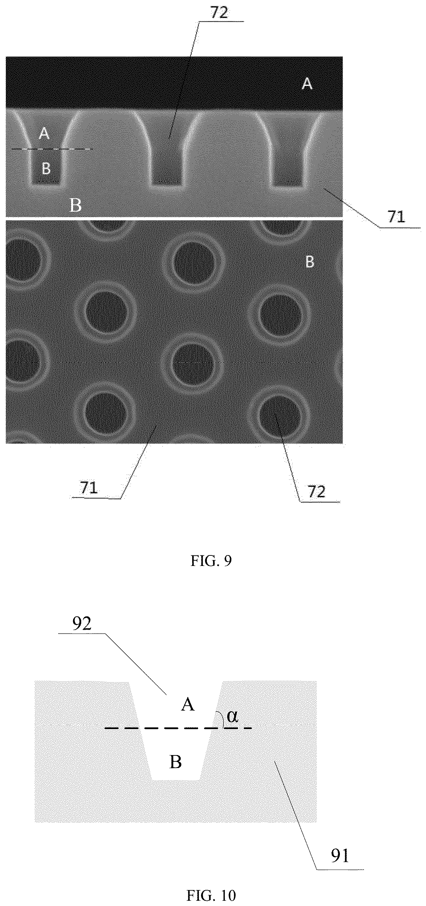

Specifically, in one embodiment, when the maximum dimension a of the depression is greater than the maximum dimension b, and the maximum dimension b is equal to the maximum dimension d, the included angle α is less than 90°, and at this point, a depression with a horn-shaped structure can be obtained, as shown in FIGs. 5-9. In some examples, for the depression with the horn-shaped structure, the first geometric structure and the second geometric structure are both symmetrical geometric structures, and the axes of symmetry of the first geometric structure and the second geometric structure coincide. Refer to FIGs. 8 and 9. FIG. 8 is a schematic longitudinal cross-sectional view of a chip substrate with the first structure, where 71 is the chip substrate, 72 is the depression, A is the first geometric structure, B is the second geometric structure; FIG. 9 is a physical diagram of a chip with the first structure, where A is the longitudinal sectional view, and B is the top view.

When the depression has the horn-shaped structure, the depth c is preferably 150 nm-5 µm, more preferably 200 nm-3 µm, and most preferably 250 nm-1 µm. The maximum dimension a is preferably 100 nm-5 µm, more preferably 150 nm-3 µm, and most preferably 200 nm-1 µm. The maximum dimension b is preferably 100 nm-5 µm, more preferably 150 nm-3 µm, and most preferably 200 nm-1 µm. The included angle α preferably satisfies the following relationship: 10° ≤ α < 90°, more preferably α = 20-80°, even more preferably α = 30-70°, and most preferably α = 40-60°. The depth e is preferably 100 nm-5 µm, more preferably 150 nm-3 µm, and most preferably 200 nm-1 µm. The maximum dimension d is preferably 100 nm-5 µm, more preferably 150 nm-3 µm, and most preferably 200 nm-1 µm.

In another embodiment, the maximum dimension a of the depression is greater than the maximum dimension b, the maximum dimension b is greater than the maximum dimension d, the maximum dimension d is not 0, and the included angle α is less than 90°. For this embodiment, it can also be understood that the first geometric structure of the depression is a geometric structure in which the inner diameter gradually decreases with increasing depth, and the depth e of the second geometric structure is 0; alternatively, it can be understood that the depth c of the first geometric structure of the depression is 0, and the second geometric structure is a geometric structure in which the inner diameter gradually decreases with increasing depth. In some examples, the first geometric structure and the second geometric structure of the depression are both symmetrical geometric structures, and the axes of symmetry of the first geometric structure and the second geometric structure coincide. Illustratively, the depression is a depression with a flat-bottomed conical structure (an inverted truncated cone structure), see FIGs. 10 and 11. FIG. 10 is a schematic longitudinal cross-sectional view of a chip substrate with the second structure, where 91 is the chip substrate, and 92 is the depression; FIG. 11 is a physical diagram of a chip with the second structure, where A is the longitudinal sectional view, and B is the top view. When the depression has the flat-bottomed conical structure, the depth c is preferably 150 nm-5 µm, more preferably 200 nm-3 µm, and most preferably 250 nm-1 µm. The maximum dimension a is preferably 100 nm-5 µm, more preferably 150 nm-3 µm, and most preferably 200 nm-1 µm. The maximum dimension b is preferably 100 nm-5 µm, more preferably 150 nm-3 µm, and most preferably 200 nm-1 µm. The included angle α preferably satisfies the following relationship: 10° ≤ α < 90°, more preferably α = 20-80°, even more preferably α = 30-70°, and most preferably α = 40-60°. The depth e is preferably 100 nm-5 µm, more preferably 150 nm-3 µm, and most preferably 200 nm-1 µm. The maximum dimension d is preferably 100 nm-5 µm, more preferably 150 nm-3 µm, and most preferably 200 nm-1 µm. Specifically, when the depression has the flat-bottomed conical structure, the value of the maximum dimension b can be disregarded, and instead, the ratio of the maximum dimension a to the maximum dimension d can be defined by the total depth (i.e., the sum of the depth c and the depth e) and the included angle α. In some specific embodiments, the sum of the depth c and the depth e is preferably 100 nm-5 µm, more preferably 150 nm-3 µm, and most preferably 200 nm-1 µm.

In yet another embodiment, the maximum dimension a of the depression is greater than the maximum dimension b, and the maximum dimension d is 0. For this embodiment, it can also be understood that the first geometric structure of the depression is a geometric structure in which the inner diameter gradually decreases with increasing depth until it reaches 0, and the depth e of the second geometric structure is 0; alternatively, it can be understood that the depth c of the first geometric structure of the depression is 0, and the second geometric structure is a geometric structure in which the inner diameter gradually decreases with increasing depth until it reaches 0. In some examples, the first geometric structure and the second geometric structure of the depression are both symmetrical geometric structures, and the axes of symmetry of the first geometric structure and the second geometric structure coincide. Illustratively, the depression is a depression with a conical structure, see FIGs. 12 and 13. FIG. 12 is a schematic longitudinal cross-sectional view of a chip substrate with the third structure, where 111 is the chip substrate, and 112 is the depression; FIG. 13 is a physical diagram of a chip with the third structure, where A is the longitudinal sectional view, and B is the top view.

When the depression has the conical structure, the depth c is preferably 150 nm-5 µm, more preferably 200 nm-3 µm, and most preferably 250 nm-1 µm. The maximum dimension a is preferably 100 nm-5 µm, more preferably 150 nm-3 µm, and most preferably 200 nm-1 µm. The maximum dimension b is preferably 100 nm-5 µm, more preferably 150 nm-3 µm, and most preferably 200 nm-1 µm. The included angle α preferably satisfies the following relationship: 10° ≤ α < 90°, more preferably α = 20-80°, even more preferably α = 30-70°, and most preferably α = 40-60°. The depth e is preferably 100 nm-5 µm, more preferably 150 nm-3 µm, and most preferably 200 nm-1 µm. Specifically, when the depression has the conical structure, the value of the maximum dimension b can be disregarded, and instead, the maximum dimension a can be defined by the total depth (i.e., the sum of the depth c and the depth e) and the included angle α. In some specific embodiments, the sum of the depth c and the depth e is preferably 100 nm-5 µm, more preferably 150 nm-3 µm, and most preferably 200 nm-1 µm.

In the embodiments of the present application, in addition to the aforementioned depression that is wider at the top and narrower at the bottom, the surface of the chip substrate may also include a depression that is narrower at the top and wider at the bottom or a depression that is consistent in width from the top to the bottom.

In one embodiment, the maximum dimension a of the depression is equal to the maximum dimension b, which is equal to the maximum dimension d; at this point, the included angle α is 90°, and the depression can be considered a depression with a cylindrical structure. For this embodiment, it can also be understood that the first geometric structure of the depression is a geometric structure with a constant inner diameter, and the depth e of the second geometric structure is 0; alternatively, it can be understood that the depth c of the first geometric structure of the depression is 0, and the second geometric structure is a geometric structure with a constant inner diameter. In some examples, the first geometric structure and the second geometric structure of the depression are both symmetrical geometric structures, and the axes of symmetry of the first geometric structure and the second geometric structure coincide. Refer to FIGs. 14 and 15. FIG. 14 is a schematic cross-sectional view of a chip substrate with the fourth structure, where 131 is the chip substrate, and 132 is the depression; FIG. 15 is a physical diagram of a chip with the fourth structure, where A is the longitudinal sectional view, and B is the top view.

When the depression has the cylindrical structure, the depth c is preferably 150 nm-5 µm, more preferably 200 nm-3 µm, and most preferably 250 nm-1 µm. The maximum dimension a is preferably 100 nm-5 µm, more preferably 150 nm-3 µm, and most preferably 200 nm-1 µm. The maximum dimension b is preferably 100 nm-5 µm, more preferably 150 nm-3 µm, and most preferably 200 nm-1 µm. The depth e is preferably 100 nm-5 µm, more preferably 150 nm-3 µm, and most preferably 200 nm-1 µm. Specifically, when the depression has the cylindrical structure, the value of the maximum dimension b can be disregarded, and instead, the morphology of the depression can be defined by the total depth (i.e., the sum of the depth c and the depth e). In some specific embodiments, the sum of the depth c and the depth e is preferably 100 nm-5 µm, more preferably 150 nm-3 µm, and most preferably 200 nm-1 µm.

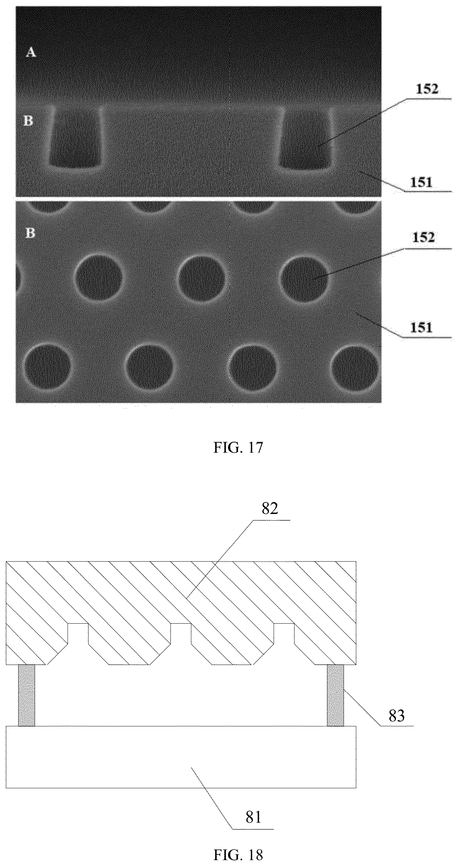

In one embodiment, the maximum dimension a of the depression is less than the maximum dimension b, and the maximum dimension b is less than the maximum dimension d; at this point, the included angle α is greater than 90°, and the depression can be considered a depression with a pear-shaped structure. For this embodiment, it can also be understood that the first geometric structure of the depression is a geometric structure in which the inner diameter gradually increases with increasing depth, and the depth e of the second geometric structure is 0; alternatively, it can be understood that the depth c of the first geometric structure of the depression is 0, and the second geometric structure is a geometric structure in which the inner diameter gradually increases with increasing depth. In some examples, the first geometric structure and the second geometric structure of the depression are both symmetrical geometric structures, and the axes of symmetry of the first geometric structure and the second geometric structure coincide. Refer to FIGs. 16 and 17. FIG. 16 is a schematic cross-sectional view of a chip substrate with the fifth structure, where 151 is the chip substrate, and 152 is the depression; FIG. 17 is a physical diagram of a chip with the fifth structure, where A is the longitudinal sectional view, and B is the top view.

When the depression has the pear-shaped structure, the depth c is preferably 150 nm-5 µm, more preferably 200 nm-3 µm, and most preferably 250 nm-1 µm. The maximum dimension a is preferably 100 nm-5 µm, more preferably 150 nm-3 µm, and most preferably 200 nm-1 µm. The maximum dimension b is preferably 100 nm-5 µm, more preferably 150 nm-3 µm, and most preferably 200 nm-1 µm. The included angle α preferably satisfies the following relationship: 90° < α ≤ 170°, more preferably α = 95-160°, even more preferably α = 100-150°, and most preferably α = 110-140°. The depth e is preferably 100 nm-5 µm, more preferably 150 nm-3 µm, and most preferably 200 nm-1 µm. Specifically, when the depression has thepear-shaped structure, the value of the maximum dimension b can be disregarded, and instead, the morphology of the depression can be defined by the total depth (i.e., the sum of the depth c and the depth e) and the included angle α. In some specific embodiments, the sum of the depth c and the depth e is preferably 100 nm-5 µm, more preferably 150 nm-3 µm, and most preferably 200 nm-1 µm.

Compared with the structure that is consistent in width from the top to the bottom or the structure that is narrower at the top and wider at the bottom (as shown in FIGs. 14-17), the micro/nano structure that is wider at the top and narrower at the bottom (as shown in FIGs. 5-13) facilitates substance exchange and diffusion between the liquid phase of a reaction reagent and active molecules on the surface of the micro/nano structure during a biochemical reaction in a detection process (e.g., nucleic acid sequencing) on the biochip, thereby promoting the reaction to occur and thus obtaining better detection signals (including signal intensity and signal-to-noise ratio) and more accurate detection results.

In some specific embodiments, the depression on the surface of the chip substrate may be one of or a combination of two or more of the aforementioned structures, preferably including at least the depression that is wider at the top and narrower at the bottom, i.e., the conical, flat-bottomed conical, or horn-shaped depression.

In some embodiments, the depressions are in a regular arrangement on the surface of the chip substrate; it can also be understood that the depressions are regularly arranged to form a pattern of a certain shape. In some examples, the surface is provided with at least one array unit having the regularly arranged depressions. Specifically, the array unit is a square array unit. That is, the pattern formed by a set of a plurality of depressions is a square. Illustratively, when the depressions are arranged in rows and columns, the distance between adjacent rows is equal to the distance between adjacent columns, and the set of the plurality of depressions includes the same number of rows and columns. The array unit is a rectangular array unit. That is, the pattern formed by a set of a plurality of depressions is a rectangle. Illustratively, when the depressions are arranged in rows and columns, the distance between adjacent rows is equal to the distance between adjacent columns, and the set of the plurality of depressions includes different numbers of rows and columns. The array unit is a honeycomb array unit. That is, the pattern formed by a set of a plurality of depressions is a honeycomb shape. In other words, the plurality of depressions are regularly arranged such that the set of depressions forms a honeycomb shape. Certainly, one surface may include two or more of the square array unit, the rectangular array unit, and the honeycomb array unit. In one example, one surface of the chip substrate may include one or more sets of depressions, and in each set, the depressions are arranged according to the same rule. In another example, one surface of the chip substrate includes one or more sets of depressions, and the arrangement of depressions in at least one set is different from that of depressions in another set. That is, one chip substrate includes a combination of multiple arrangements, e.g., by splicing or nesting.

In some embodiments, the chip may further include an encapsulation layer arranged on the chip substrate and configured for encapsulating the surface of the chip substrate provided with the depression, so as to protect the chip substrate and form a reaction region through which a solution may flow.

In one embodiment, the chip includes a first cover plate bonded to the surface of the chip substrate provided with the depression. The first cover plate is made of a material having a light transmittance of 95% or greater, such that light can reach the surface of the chip substrate provided with the depression through the first cover plate. The material of the first cover plate may be identical to or different from that of the chip substrate, which is not particularly limited in the present application. Illustratively, the material of the cover plate may be glass, quartz, a polymer film, etc.

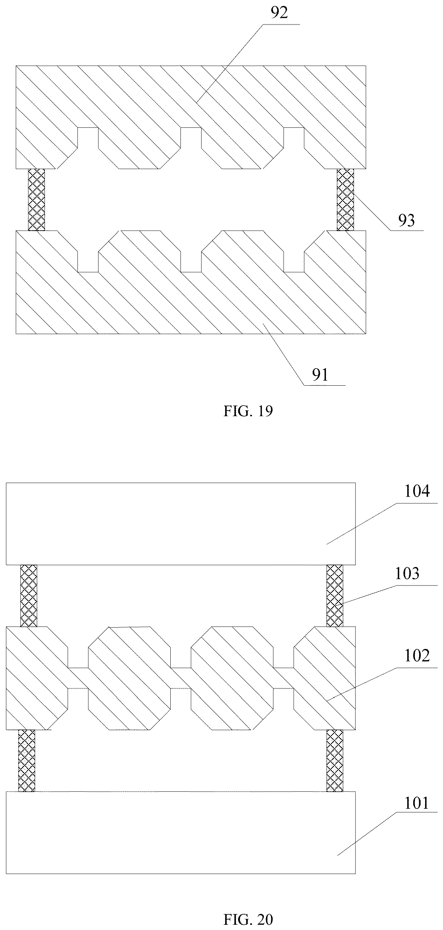

In some examples, as shown in FIGs. 18 and 19, the depression of the chip substrate 82 is formed on one surface of the chip substrate 82, and the first cover plate 81 is bonded to the surface of the chip substrate 82 provided with the depression, so as to implement the encapsulation of the chip. The first cover plate 81 may be a patterned material or an unpatterned material.

In one example, as shown in FIG. 18, the first cover plate 81 is a normal cover plate without a depression, that is, the two surfaces of the cover plate are untreated flat surfaces. At this point, the surface of the first cover plate 81 facing the chip substrate 82 is a flat surface. In one example, the glue layer 83 is arranged between the first cover plate 81 and the chip substrate 82 for bonding the first cover plate 81 and the chip substrate 82, and the glue layer 83 has a channel arranged in a length direction of the chip; the depression is arranged on the surface of the chip substrate 82 in a region where the channel is located, and the shapes and/or arrangements of the depressions in different channels on the same surface are identical or different. In some other examples, as shown in FIG. 19, the first cover plate 91 is provided with regularly arranged depressions on one surface. The depression of the first cover plate 91 is arranged opposite to the depression of the chip substrate 92. That is, the surface of the first cover plate 91 facing the chip substrate 92 is provided with regularly arranged depressions. At this point, one chip is provided with two surfaces for sequencing, thus improving the sequencing throughput. In this example, the depression of the first cover plate 91 may be identical to or different from that of the chip substrate 92. The details are not recited here for brevity. In one example, the glue layer 93 is arranged between the first cover plate 91 and the chip substrate 92 for bonding the first cover plate 91 and the chip substrate 92, and the glue layer 93 has a channel arranged in a length direction of the chip; the depression is arranged on the surfaces of the chip substrate 92 and the first cover plate 91 in a region where the channel is located, and the shapes and/or arrangements of the depressions in different channels on the same surface are identical or different.

In another embodiment, the chip substrate is provided with regularly arranged depressions on both surfaces, and the depressions on the two surfaces may be in identical or different arrangements. The arrangement of the depressions on the two surfaces of the chip substrate has been described above and is not recited here for brevity. At this point, the light transmittance of the chip substrate is less than 70% or the thickness of the chip substrate is 500 µm or greater, such that when an imaging apparatus collects the optical signals from the two surfaces (especially the depressions) of the chip substrate, the interference between the optical signals of the two surfaces is reduced. In this embodiment, the cover plate is bonded to at least one surface of the chip substrate provided with the depression, so as to implement the encapsulation of the chip. In some examples, the chip includes a first cover plate and a second cover plate respectively bonded to the two surfaces of the chip substrate, where the first cover plate and the second cover plate are made of a material having a light transmittance of 95% or greater, such that light can reach the two surfaces of the chip substrate provided with the depression through the first cover plate and the second cover plate, respectively.

In some examples, the cover plates are normal cover plates without a depression, that is, the two surfaces of the cover plates are untreated flat surfaces. In one example, as shown in FIG. 20, the first cover plate 101 and the second cover plate 104 are respectively bonded to the two surfaces of the chip substrate 102 provided with the depression, and the first cover plate 101 and the second cover plate 104 are normal cover plates without a depression, that is, the two surfaces of the first cover plate 101 and the second cover plate 104 are flat surfaces. In one example, the glue layers 103 are arranged between the first cover plate 101 and the chip substrate 102 and between the second cover plate 104 and the chip substrate 102 for bonding the first cover plate 101 and the chip substrate 102, and the second cover plate 104 and the chip substrate 102. The glue layers 103 have channels arranged in a length direction of the chip; the depressions are arranged on the surfaces of the chip substrate 102 in regions where the channels are located, and the shapes and/or arrangements of the depressions in different channels on the same surface are identical or different.

In some examples, the first cover plate and/or the second cover plate are cover plates provided with the depression on one surface.

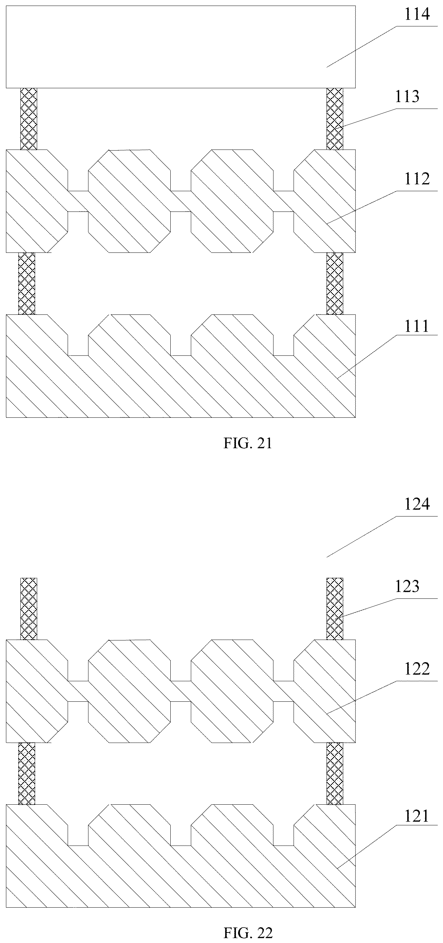

In one example, as shown in FIG. 21, the first cover plate 111 and the second cover plate 114 are respectively bonded to the two surfaces of the chip substrate 112 provided with the depression, where the first cover plate 111 is a cover plate provided with the depression, and the depression of the first cover plate 111 is arranged opposite to the depression of the chip substrate 112; the second cover plate is a normal cover plate without a depression, and the two surfaces of the second cover plate 114 are flat surfaces. It should be understood that the depression of the first cover plate 111 may be identical to or different from that of the chip substrate 112. In one example, the glue layers 113 are arranged between the first cover plate 111 and the chip substrate 112 and between the second cover plate 114 and the chip substrate 112 for bonding the first cover plate 111 and the chip substrate 112, and the second cover plate 114 and the chip substrate 112. The glue layers 113 have channels arranged in a length direction of the chip; the depressions are arranged on the surfaces of the chip substrate 112 and the first cover plate 111 in regions where the channels are located, and the shapes and/or arrangements of the depressions in different channels on the same surface are identical or different.

In one example, as shown in FIG. 22, the first cover plate 121 and the second cover plate 124 are respectively bonded to the two surfaces of the chip substrate 122 provided with the depression, where the two cover plates are cover plates provided with the depression, and the depressions of the cover plates are arranged opposite to the depression of the chip substrate 122 to form a double-chip-like, laminated structure. It should be understood that the depression of each cover plate may be identical to or different from that of the chip substrate; the depressions of the two cover plates may be identical or different. In one example, the glue layers 123 are arranged between the first cover plate 121 and the chip substrate 122 and between the second cover plate 124 and the chip substrate 122 for bonding the first cover plate 121 and the chip substrate 122, and the second cover plate 124 and the chip substrate 122. The glue layers 123 have channels arranged in a length direction of the chip; the depressions are arranged on the surfaces of the chip substrate 122, the first cover plate 121, and the second cover plate 122 in regions where the channels are located, and the shapes and/or arrangements of the depressions in different channels on the same surface are identical or different.

When the chip is used for sequencing, by arranging the regularly arranged depressions on the surface of the cover plate facing the chip substrate, the above embodiment can increase the reaction and/or detection sites for nucleic acid molecules on the surface of the chip, which is beneficial to increasing the sequencing throughput. It should be understood that when the cover plate is provided with the regularly arranged depressions, the reaction and/or detection sites for the nucleic acid molecules on the surface of the chip can be increased, thus facilitating the improvement of the sequencing throughput. Such a cover plate also possesses the functionality of the chip substrate, and thus may also be referred to as a chip substrate.

In some embodiments, the chip includes a polymer layer arranged on the surface provided with the depression. Since the chip substrate is provided with the depression on at least one surface, the polymer layer is arranged at least on the surface of the chip substrate provided with the depression. When the two surfaces of the chip substrate are provided with the depression, both surfaces are provided with the polymer layer. Similarly, when the cover plate, including the first cover plate and the second cover plate, is provided with the depression on one surface, the polymer layer is correspondingly arranged on the surface of the cover plate provided with the depression. In this embodiment, the surface of the depression includes the side wall and the bottom surface of the depression. When the depression has no bottom surface, for example, when the depression has the conical structure, the surface of the depression is the wall of the depression; at this point, the polymer layer is arranged on the bottom surface of the depression structure.

The polymer layer provides a capture site or a detection site for the analysis and/or detection of nucleic acid molecules by binding to a ligand for nucleic acid molecules, such as a nucleic acid probe, an antigen/antibody, a nucleic acid-protein complex, etc. As such, in the examples of the present application, the shape, dimensions, and arrangement of the depression are designed to ensure that the nucleic acid molecules are immobilized on the surface of the chip in a certain arrangement density and arrangement manner. That is, the nucleic acid molecules form the detection or analysis sites in a certain density and arrangement manner, which is beneficial to acquiring and positioning the information generated during the reaction and/or detection of the nucleic acid molecules in the later stage, and is helpful to improve the stability of the detection results for the nucleic acid molecules.

In some examples, the polymer layer is formed from a polymer containing a first functional group selected from one or more of sulfydryl, formyl, hydroxyl, carboxyl, amino, alkenyl, alkynyl, and azido. These functional groups are chemically reactive or adsorptive, and can react with other functional groups with reactivity to bind to a nucleic acid molecule or a ligand for nucleic acid molecules, such as a nucleic acid probe, an antigen/antibody, or a nucleic acid-protein complex. Illustratively, the polymer constituting the polymer layer may be, but is not limited to, polyethylene glycol, polypropylene, polyacrylic acid, poly(methyl methacrylate), etc. In the examples of the present application, the polymer formed on the surface of the chip provided with the depression can optimize the environment for the chip sequencing reaction. Particularly, the formed polymer layer has a high transparency and a low optical background in a visible light wave band of 400 nm-700 nm.

The thickness of the polymer layer is not particularly limited in the present application, as long as the nucleic acid molecule or the ligand for nucleic acid molecules can be immobilized.

In this embodiment, since the material of the chip substrate and/or the cover plate are less reactive, in some examples, the polymer layer is bonded to the surface of the chip substrate and/or the cover plate via a coupling molecule. In one example, the coupling molecule has the following molecular formula: XₐSi(Y_{b})M_{c}Z, where X and Z are reactive groups for coupling the functional groups of the chip substrate and the polymer, respectively.

Among these, X is H, F, Cl, Br, I, C₁₋₆ alkyl, C₁₋₆ haloalkyl, or C₁₋₆ alkoxy, for example, Cl, CH₃O-, CH₃CH₂O-, etc.; Y is selected from C₁₋₁₀ alkyl, e.g., CH₃-, CH₃CH₂-, etc.; M_{c} is selected from C₁₋₅₀ alkylene, -(OCH₂CH₂)_{c}-, and -(CH(OH)CH₂-)_{c}; Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl; the C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl are substituted with at least one R^{a}, and each R^{a} is independently selected from halogen, nitro, ester group, amino, carboxyl, hydroxyl, azido, and epoxy group; a, b, and c are integers meeting the following conditions: 0 ≤ a ≤ 3, 0 ≤ b ≤ 3, and a + b = 3; 1 ≤ c ≤ 50.

Illustratively, the coupling molecule may be at least one of the following structures: 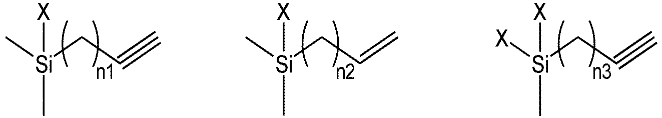 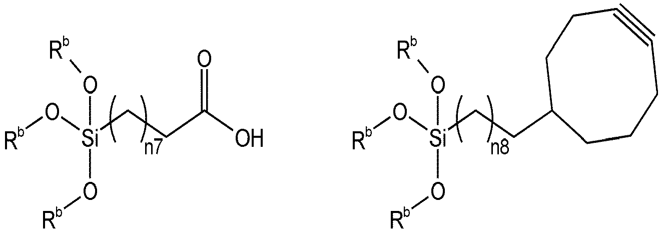 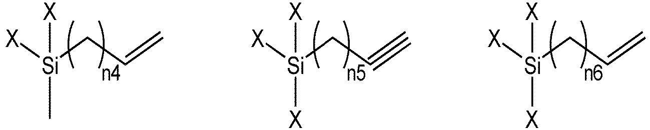 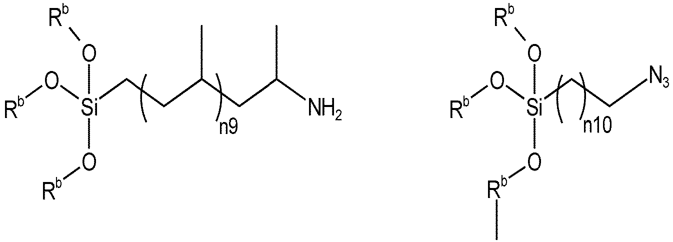
where X is halogen; illustratively, X is F, Cl, Br, or I;
R^{b} is -CH₃, -C₂H₅, -C₃H₇, or -C₄H₉;
n1 is selected from integers of 2-10; illustratively, n1 is 2, 3, 4, 5, 6, 7, 8, 9, or 10;
n2 is selected from integers of 2-10; illustratively, n2 is 2, 3, 4, 5, 6, 7, 8, 9, or 10;
n3 is selected from integers of 2-10; illustratively, n3 is 2, 3, 4, 5, 6, 7, 8, 9, or 10;
n4 is selected from integers of 2-10; illustratively, n2 is 2, 3, 4, 5, 6, 7, 8, 9, or 10;
n5 is selected from integers of 2-10; illustratively, n2 is 2, 3, 4, 5, 6, 7, 8, 9, or 10;
n6 is selected from integers of 2-10; illustratively, n2 is 2, 3, 4, 5, 6, 7, 8, 9, or 10;
n7 is selected from integers of 1-10; illustratively, n2 is 1, 2, 3, 4, 5, 6, 7, 8, 9, or 10;
n8 is selected from integers of 5-10; illustratively, n2 is 5, 6, 7, 8, 9, or 10;
n9 is selected from integers of 1-10; illustratively, n2 is 1, 2, 3, 4, 5, 6, 7, 8, 9, or 10;
n10 is selected from integers of 1-10; illustratively, n2 is 1, 2, 3, 4, 5, 6, 7, 8, 9, or 10.

In some embodiments, the chip further includes a first film layer arranged between the chip substrate and/or the cover plate and the polymer layer. Specifically, the first film layer is optionally arranged on the surfaces of the chip substrate provided with the depression and the cover plate provided with the depression, and the material of the first film layer is different from that of the surface bonded thereto, so as to reduce surface defects of the chip substrate and/or the cover plate and improve the uniformity of the surface of the chip. Furthermore, in the preparation process of the chip, considering the feasibility of the process, it is a tendency to form the polymer layer by reaction on the entire surface of the chip substrate and/or the cover plate and then treat the polymer layer on the surfaces of the chip substrate and/or the cover plate to retain only the polymer formed in the depressions, so as to meet the requirement of detecting the optical signal generated by the reaction of the nucleic acid molecules. In some examples, the polymer is coupled within the depression by a selective modification process. In some examples, the polymer outside the depression is removed by a polishing process. At this point, the polishing endpoint may be determined by arranging the first film layer on the surface of the chip substrate and/or the cover plate, and indicating the degree of polishing reaction by detecting the physicochemical properties of the first film layer.

In this embodiment, the first film layer includes, but is not limited to, a metal film, such as Al film, Au film, etc.; a metal oxide film, such as Al₂O₃ film, etc.; an inorganic oxide film, such as SiO₂ film, etc. The first film layer may be one or more of the above, preferably one or more of Al film, Al₂O₃ film, and SiO₂ film. Illustratively, when the SiO₂ film is used as the first film layer, the SiO₂ film can stabilize the properties of the surface of the chip, and specifically, avoid the reaction between materials, reagents or the like introduced by the reaction and/or detection with the chip substrate and thus the generation of bubbles. In addition, the SiO₂ can also improve the bonding firmness of the polymer and thus reduce the risk of the polymer layer falling off from the surface of the chip.

In some examples, the first film layer has a thickness of 10-1000 nm, preferably 15-800 nm, more preferably 20-500 nm, and most preferably 20-200 nm. Within this thickness range, the first film layer can exert the above effects. For the first film layer having an excessive thickness, e.g., greater than 1500 nm, the light intensity of the base collected from the depression of the chip provided with the first film layer is reduced during the sequencing as compared to the chip without the first film layer, thereby affecting the determination of the sequencing signals. Illustratively, the thickness of the first film layer may be 10 nm, 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 110 nm, 120 nm, 130 nm, 140 nm, 150 nm, 160 nm, 170 nm, 180 nm, 190 nm, 200 nm, 250 nm, 300 nm, 400 nm, 500 nm, 600 nm, 700 nm, 800 nm, 900 nm, 1000 nm, etc.

In some examples, the chip substrate and the cover plate are bonded by an adhesive material, and the adhesive material forms a channel structure through hollowing to provide a reaction region for the chip substrate. The adhesive material may be a binder, a double-sided tape, or a light-curable material such as a UV glue, etc. Considering the influence on the reagents in the sequencing process, a binder that has no influence on the nucleic acid molecules and the reagents used in the sequencing process and remains stable in the reagents used in the sequencing process should be selected. Illustratively, the adhesive material is a UV-curable adhesive material, a thermosetting adhesive material, a pressure-sensitive adhesive material, etc, which, however, is not limited thereto.

In some examples, the chip has at least one channel arranged in a length direction, for example, by forming one or more channels between the chip substrate and the encapsulation layer, to achieve fluid communication functionality. Specifically, the depression is arranged on the surface of the chip substrate in a region where the channel is located, and the solution reacts with the nucleic acid molecules immobilized on the depression when flowing through the channel. It should be understood that the shapes and/or arrangements of the depressions in different channels are identical or different.

By forming the regularly arranged depressions on the substrate, the chip provided by the examples of the present application can improve the stability of sequencing and the accuracy of sequencing results. Moreover, the regularly arranged depressions can improve the arrangement density of the depressions on the chip and thus the detection throughput; the detection sites corresponding to the depressions can be positioned through the regularly arranged depressions, thereby facilitating the synchronous detection of multiple types of nucleic acid molecules on the surface of the same chip.

The chip provided by the examples of the present application may be prepared by the following method, which, however, is not limited thereto.

Correspondingly, the examples of the present application further provide a method for preparing a chip, including the following step:
a) forming regularly arranged depressions on at least one surface of a substrate, where the depression includes, in sequence, a first geometric structure and a second geometric structure that are interconnected in a direction from a surface of a chip substrate toward a central part of the chip substrate; the first geometric structure has a depth c of 0-100 µm in a direction perpendicular to the surface of the chip, a maximum dimension a of 10 nm-100 µm in a direction parallel to the surface of the chip, and a maximum dimension b of 10 nm-100 µm in a horizontal direction on the side away from the surface of the chip; an included angle α between a side wall of the first geometric structure and the horizontal direction on the side away from the surface of the chip is 10-170°;
   the second geometric structure has a depth e of 0-100 µm in the direction perpendicular to the surface of the chip and a maximum dimension d of 0-100 µm in the horizontal direction on the side away from the surface of the chip;
   the depth c and the depth e are not simultaneously 0.

In step a), the substrate is a target substrate requiring the arrangement of the depression and includes at least a chip substrate. In some examples, the target substrate requiring the arrangement of the depression is the chip substrate. In some examples, the target substrate requiring the arrangement of the depression is a cover plate, and at this point, the substrate is the cover plate. In some examples, the target substrate requiring the arrangement of the depression includes a chip substrate and a cover plate, and at this point, the substrate includes the chip substrate and the cover plate. The materials of the substrate can be selected according to the above materials of the chip substrate and the cover plate, which are not recited here.

In the examples of the present application, the regularly arranged depressions are prepared on the surface of the substrate, and the shape, dimension parameter, and arrangement rule of the depression are as described above, which are not recited here.

In the examples of the present application, the regularly arranged depressions can be prepared on the surface of the substrate by means of photoetching technology, nano-imprinting technology, screen printing technology, etc.

Illustratively, the preparation of the depression by photoetching technology includes the following steps: cleaning and drying the substrate, applying a photoresist, and exposing, developing and etching to give the regularly arranged depressions, i.e., to directly acquire a pattern. The preparation may also include the following steps: applying a photoresist on another substrate, exposing, developing and etching to give the regularly arranged depressions, and transferring the depressions to a target substrate to form a pattern. The reagents, methods, and the like for photoetching are not particularly limited in the examples of the present application, where the method for etching may be dry etching, such as plasma etching, electron beam etching, laser etching, etc., and may also be wet etching, such as acid or base etching, oxidation-reduction etching, etc.

Illustratively, the preparation of the depression by nano-imprinting technology may include the following steps: preparing a template of a required pattern, applying a photoresist on the substrate, pressing the template on the surface of the photoresist, and transferring the pattern onto the photoresist by pressurizing to give the regularly arranged depressions, i.e., to directly acquire the pattern. The preparation may also include the following steps: preparing a template of a required pattern on a substrate, applying a photoresist on a second substrate, pressing the template on the surface of the photoresist, transferring the pattern onto the photoresist by pressurizing, curing the photoresist to give the regularly arranged depressions, and transferring the depressions onto the chip substrate by etching to form the pattern. The reagents, methods, and the like for nano-imprinting technology are not particularly limited in the examples of the present application, where the method for etching may be dry etching, such as plasma etching, electron beam etching, laser etching, etc., and may also be wet etching, such as acid or base etching, oxidation-reduction etching, etc.

Illustratively, the preparation of the depression by screen printing technology specifically includes the following step: preparing the regularly arranged depressions on the chip substrate by screen printing, i.e., to directly acquire a pattern. The preparation may also include the following steps: preparing the regularly arranged depressions on a substrate by screen printing, and transferring the depressions onto the chip substrate by etching to form the pattern. The reagents, methods, and the like for screen printing technology are not particularly limited in the examples of the present application, where the method for etching may be dry etching, such as plasma etching, electron beam etching, laser etching, etc., and may also be wet etching, such as acid or base etching, oxidation-reduction etching, etc.

In some embodiments, the pattern may be produced by one of or a combination of two or more of the methods described above. After the depression is acquired, it may be post-treated to give the final patterned surface. In some specific embodiments, the post-treatment includes, but is not limited to, an enhancement treatment, such as physical vapor deposition (PVD), chemical vapor deposition (CVD), electroplating, etc.; or a removal treatment, such as plasma ashing, solution etching, dry oxidation, etc. The specific process parameters of the above treatment are not particularly limited in the examples of the present application, and can be selected by those skilled in the art as needed.

In some embodiments, the method for preparing the chip further includes: preparing a first film layer on the surface of the chip substrate provided with the depression, where the first film layer and the substrate are made of different materials. The parameters and materials of the first film layer and the substrate are as described above, which are not recited here. In some examples, the first film layer is selected from one or more of a metal film, a metal oxide film, and an inorganic oxide film. In some examples, the first film layer is made of at least one of Au, Al, and SiO₂. In some examples, the first film layer has a thickness of 10-1000 nm.

In the examples of the present application, the first film layer may be formed on the substrate by deposition before the depression is prepared; the first film layer may also be deposited after the depression is prepared and formed on the chip substrate. That is, the first film layer may be prepared before step a) or after step a). The deposition method is not particularly limited in the examples of the present application, and may be physical vapor deposition (PVD), chemical vapor deposition (CVD), electroplating, oxidation, etc.

In some specific embodiments, the method further includes the following step after forming the regularly arranged depressions:
b) forming a polymer layer in the depression.

After the regularly arranged depressions are formed on the substrate, the surface is modified to provide capture or detection sites.

In some examples, the chip substrate is preferably pre-treated before forming the polymer layer. In some specific embodiments, the pre-treatment includes cleaning and plasma activation. The cleaning may be a solution cleaning, followed by plasma activation. In some specific embodiments, the gas used for plasma activation includes, but is not limited to, air, nitrogen, argon, hydrogen, carbon tetrafluoride, etc., and may be one of or a mixture of two or more of these gases. In some specific embodiments, the power of the plasma activation is 10 W-2 kW.

In one embodiment, the depression is a depression formed on the surface of the chip substrate and/or the cover plate, and at this point, b) includes:
bonding a polymer containing a first functional group to the surface provided with the depression; and
polishing the polymer to remove the polymer outside the region where the depression is located, so as to form the polymer layer on the depression.

It should be understood that this embodiment includes the case where the polymer is bonded to the surface provided with the depression via a coupling molecule. In this way, the polymer at the periphery of the depression can be removed to reduce the nonspecific adsorption on the surface of the chip.

In the examples of the present application, the polymer is polished to remove the polymer outside the region where the depression is located, where the removal method is not particularly limited, and includes but is not limited to solution cleaning, chemical polishing, mechanical polishing, chemical mechanical polishing, etc. Specifically, the solution cleaning is to remove the polymer molecules at the periphery of the depression by using a reagent capable of dissolving and removing the polymer molecules, such as an acid, a base, a solvent, a surfactant, etc., and is preferably performed under ultrasonic conditions or performed by using a flushing manner. In some embodiments, the polymer is removed by polishing, which may be a chemical polishing based on chemical reaction removal, a mechanical polishing based on mechanical action removal, or a combination thereof. In the examples of the present application, the polishing may be performed by using chemical polishing reagents, such as one or more of an acid, a base, an organic solvent, and a surfactant, or may be a mechanical polishing using, e.g., one or more reagents of diamond, silica sol, calcium carbonate, cerium oxide, zirconium oxide, etc., as the polishing reagent.

In the technical schemes provided by the present application, when the substrate is treated by polishing, the chip substrate is preferably a structure including the substrate and the first film layer. At this point, the first film layer can be used as a polishing endpoint indicator for indicating the endpoint of polishing, thus facilitating the control of the polishing on the surface to give a chip with a stable and uniform surface, and thus further facilitating the detection stability.

In another embodiment, the depression is a protrusion formed on the surface of the chip substrate and/or the cover plate, and at this point, a) includes: bonding the polymer containing the first functional group to the surface of the substrate. The substrate with the polymer formed on the surface is then patterned to give the regularly arranged depressions on the surface.

According to the above two embodiments, in some examples, the first functional group is selected from one or more of sulfydryl, formyl, hydroxyl, carboxyl, amino, alkenyl, alkynyl, and azido.

According to the above two embodiments, in some examples, the substrate is treated with the coupling molecule, such that the coupling molecule is formed on the surface of the substrate to be treated or the surface provided with the depression, and the coupling molecule is modified with the polymer to form the polymer layer. The chip substrate is preferably pre-treated before the coupling molecule is bonded to the surface of the chip substrate, and in some specific embodiments, the pre-treatment includes cleaning and plasma activation. The cleaning may be a solution cleaning, followed by plasma activation. In some specific embodiments, the gas used for plasma activation includes, but is not limited to, air, nitrogen, argon, hydrogen, carbon tetrafluoride, etc., and may be one of or a mixture of two or more of these gases. In some specific embodiments, the power of the plasma activation is 10 W-2 kW. After the pre-treatment, the coupling molecule can be bonded by a method such as chemical vapor deposition (CVD), physical vapor deposition (PVD), atomic layer deposition, magnetron sputtering, a solution-based method, spin coating, spraying, etc., so as to provide active sites for immobilizing the polymer.

The coupling molecule has the following molecular formula: XₐSi(Y_{b})M_{c}Z, where X and Z are reactive groups for coupling the functional groups of the chip substrate and the polymer, respectively.

Among these, X is H, F, Cl, Br, I, C₁₋₆ alkyl, C₁₋₆ haloalkyl, or C₁₋₆ alkoxy, for example, Cl, CH₃O-, CH₃CH₂O-, etc.; Y is selected from C₁₋₁₀ alkyl, e.g., CH₃-, CH₃CH₂-, etc.; M_{c} is selected from C₁₋₅₀ alkylene, -(OCH₂CH₂)_{c}-, and -(CH(OH)CH₂-)_{c}; Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl; the C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl are substituted with at least one R^{a}, and each R^{a} is independently selected from halogen, nitro, ester group, amino, carboxyl, hydroxyl, azido, and epoxy group; a, b, and c are integers meeting the following conditions: 0 ≤ a ≤ 3, 0 ≤ b ≤ 3, and a + b = 3; 1 ≤ c ≤ 50.

In some possible embodiments, the polymer may be bonded to the coupling molecule by a solution-based method, spin coating, screen printing, spraying, etc. In some specific embodiments, the temperature for modifying the coupling molecule with the polymer to form the polymer layer is 25-100 °C and the time is 1-48 h.

In some examples, after forming the polymer layer, the polymer and coupling molecules at the periphery of the depression are removed by post-treatment to reduce the non-specific adsorption on the surface of the biochip. In the examples of the present application, the removal method is not particularly limited, and includes but is not limited to solution cleaning, chemical polishing, mechanical polishing, chemical mechanical polishing, etc. Specifically, the solution cleaning is to remove the coupling molecules at the periphery of the depression by using a reagent capable of dissolving and removing the coupling molecules, such as an acid, a base, a solvent, a surfactant, etc., and is preferably performed under ultrasonic conditions or performed by using a flushing manner. In some embodiments, the coupling molecule is removed by polishing, which may be a chemical polishing based on chemical reaction removal, a mechanical polishing based on mechanical action removal, or a combination thereof. In the examples of the present application, the polishing may be performed by using chemical polishing reagents, such as one or more of an acid, a base, an organic solvent, and a surfactant, or may be a mechanical polishing using, e.g., one or more reagents of diamond, silica sol, calcium carbonate, cerium oxide, zirconium oxide, etc., as the polishing reagent.

Similarly, when the chip substrate is treated by polishing, the chip substrate is preferably a structure including the chip substrate and the first film layer. At this point, the first film layer can be used as a polishing endpoint indicator for indicating the endpoint of polishing, thus facilitating the control of the polishing on the surface to evenly polish the surface and modify the coupling molecule, giving a chip with a stable and uniform surface, and further facilitating the detection stability.

In some specific embodiments, the method for preparing the chip further includes modifying the polymer layer with a capture molecule or a detection molecule or the like, such as a nucleic acid probe, an antigen/antibody, or a nucleic acid-protein complex. In the examples of the present application, the method for modification with the capture molecule or the detection molecule is not particularly limited, for example, by chemical coupling, including hydroxyl-carboxyl coupling, amino-carboxyl coupling, sulfydryl-sulfydryl coupling, click chemistry coupling, etc. In some specific embodiments, the modification with the capture molecule or the detection molecule may be performed after the post-treatment; alternatively, the modification with the capture molecule or the detection molecule may be performed after forming the polymer layer, and then the extra polymer, capture molecule, or detection molecule may be removed by post-treatment.

In the examples of the present application, the chip includes a chip substrate, and at least the chip substrate is prepared by the method described above. In some specific embodiments, the method further includes: encapsulating the prepared chip substrate, and forming one or more flow channels on the depression of the chip substrate to achieve fluid communication functionality, so as to achieve the flow reaction of different reagents on the chip in the detection processes and the resistance to multiple cycles of reagent scouring. In some specific embodiments, the encapsulation material may be bonded to the chip substrate by a method including, but not limited to, thermal bonding, laser bonding, chemical-physical bonding, etc., where the chemical-physical bonding may be performed by bonding with a UV-curable adhesive material, a thermosetting adhesive material, a pressure-sensitive adhesive material, etc. In some specific embodiments, the encapsulation layer may be a cover plate provided with a depression, which may be identical to or different from the depression of the chip substrate, or may be an unpatterned cover plate.

In one example, as shown in FIG. 18, the first cover plate is a normal cover plate without a depression, that is, the two surfaces of the first cover plate are untreated flat surfaces. The first cover plate is bonded to the surface of the chip substrate provided with the depression, and in the bonding process, a fluid channel allowing a fluid to flow through the depression is formed in the length direction of the surface of the chip provided with the depression.

In one example, as shown in FIG. 19, the first cover plate is a cover plate provided with regularly arranged depressions on one surface, and the depression of the first cover plate is arranged opposite to the depression of the chip substrate. At this point, the surface of the first cover plate provided with the geometric structure is bonded opposite to the surface of the chip substrate provided with the depression, and in the bonding process, a fluid channel allowing a fluid to flow through the depression is formed in the length direction of the surface of the chip provided with the depression. In this example, the depression of the first cover plate may be identical to or different from that of the chip substrate. The details are not recited here for brevity. In one example, as shown in FIG. 20, the first cover plate and the second cover plate are respectively bonded to the two surfaces of the chip substrate provided with the depression, and the first cover plate and the second cover plate are normal cover plates without a depression, that is, the two surfaces of the first cover plate and the second cover plate are flat surfaces. The first cover plate and the second cover plate are respectively bonded to the two surfaces of the chip substrate provided with the depression, and in the bonding process, a fluid channel allowing a fluid to flow through the depression is formed in the length direction of the surface of the chip provided with the depression.

In one example, as shown in FIG. 21, the first cover plate and the second cover plate are respectively bonded to two surfaces of the chip substrate provided with the depression, where the first cover plate is a cover plate provided with the depression, and the depression of the first cover plate is arranged opposite to the depression of the chip substrate; the second cover plate is a normal cover plate without a depression, and the two surfaces of the second cover plate are flat surfaces. At this point, the surface of the first cover plate provided with the geometric structure is bonded opposite to the surface of the chip substrate provided with the depression, and the second cover plate is separately bonded to the other surface of the chip substrate provided with the depression; in the bonding process, fluid channels allowing a fluid to flow through the depression are formed in the length direction of the surface of the chip provided with the depression. It should be understood that the depression of the first cover plate may be identical to or different from that of the chip substrate.

In one example, as shown in FIG. 22, the first cover plate and the second cover plate are respectively bonded to the two surfaces of the chip substrate provided with the depression, where the two cover plates are cover plates provided with the depression, and the depressions of the cover plates are arranged opposite to the depression of the chip substrate to form a double-chip-like, laminated structure. At this point, the surface of the first cover plate provided with the geometric structure is bonded opposite to the surface of the chip substrate provided with the depression, and the surface of the second cover plate provided with the depression is bonded opposite to the other surface of the chip substrate provided with the depression; in the bonding process, fluid channels allowing a fluid to flow through the depression are formed in the length direction of the surface of the chip provided with the depression. It should be understood that the depression of each cover plate may be identical to or different from that of the chip substrate; the depressions of the two cover plates may be identical or different.

When the cover plate is a cover plate provided with the depression, the cover plate may be prepared by the method described above. The material of the cover plate may be identical to or different from that of the chip substrate, and, as needed, includes, but is not limited to: glass; quartz; a silicon-based material, such as silicon dioxide, polycrystalline silicon, monocrystalline silicon, etc.; and a polymer, such as HMDS, PE, PP, etc. For various cases, reference may be made to the description above regarding the chip. In some specific embodiments, the encapsulation may be performed between any of the steps of forming the coupling molecule, forming the polymer layer, post-treatment, and modification with the detection molecule or the capture molecule, without affecting subsequent procedures.

In some examples, the above steps are performed on a wafer-like chip substrate to improve production efficiency. Therefore, the chip may be cut to give chips of a desired size before encapsulation.

After the biochip is acquired, the biochip can be used for gene sequencing, providing improved detection throughput and stability and reliability of the sequencing structure.

Specifically, the nucleic acid molecule immobilized in the depression of the chip is amplified to give an amplification cluster including 10-100000 copies of DNA molecules, and then gene sequencing is performed on the amplification cluster. Since the depression limits the amplification cluster in a certain space, the amplification cluster can present a certain shape, density and light intensity meeting the detection requirements, thereby improving the detection throughput and the stability and reliability of the sequencing structure. Moreover, by limiting the amplification cluster in the depression of the chip provided by the examples of the present application, the stability of the amplification cluster in the sequencing process can be improved, allowing the amplification cluster to endure liquid environments including acidic reagents, basic reagents, organic solvents and the like, a broad reaction temperature range of 4-90 °C, 100 or more cycles of solution scouring, etc., and to maintain good stability in such environments. Furthermore, the depression, when used as the sequencing site, does not influence the biochemical functionality of biomolecules such as polymerase, amplification enzyme and the like, and can reduce the adsorption of fluorescent dye and nucleic acid molecules.

In this example, the method for amplifying the immobilized nucleic acid molecules in the depression of the chip is not strictly limited, and surface-based polymerase chain reaction (PCR) amplification such as bridge amplification, template-walking amplification and the like, as well as rolling circle amplification, may be used, which, however, is not limited thereto.

The patterned biochip and the method for preparing same provided by the present application are further described below with reference to the following examples.

### Example 1

This example provides a patterned biochip, which includes a silicon chip substrate, where depressions are formed in a honeycomb arrangement on the silicon chip substrate; that is, the depressions are arranged in rows and columns; the depressions of two adjacent rows are not located in the same column, and the depressions of alternate rows are located in the same column; the depressions of two adjacent columns are not located in the same row, and the depressions of alternate columns are located in the same row; the depression has a pear-shaped structure, as shown in FIG. 16; it can be considered that the depression includes a first geometric structure A that is symmetrical, where the bottom diameter of the first geometric structure A in a direction parallel to the surface of the chip is smaller than its bottom diameter b in a horizontal direction on the side away from the surface of the chip, forming a shape that is narrower at the top and wider at the bottom. For the pear-shaped depression, the depth c in a direction perpendicular to the surface of the chip, the bottom diameter a in the direction parallel to the surface of the chip, the bottom diameter b in the horizontal direction on the side away from the surface of the chip, the included angle α between a side wall and the horizontal direction on the side away from the surface of the chip, and the distance f (i.e., center-to-center distance) between adjacent depressions are shown in Table 1. Table 1 shows the dimensional parameters of the depressions in the biochips provided in Examples 1-5.

A silane molecular film layer with an amino functional group and a polymer layer are sequentially formed on the surface of each depression.

The surface of the silicon chip substrate is encapsulated with a cover plate with an untreated surface.

The preparation process for the biochip was as follows: A patterned adhesive layer was first formed on the surface of a silicon chip substrate by means of nano-imprinting, and the chip substrate was then etched according to the pattern. After the adhesive layer was removed, regularly arranged depressions were formed on the silicon chip substrate. After the patterned chip substrate was formed, a silane molecular film layer with an amino functional group was formed in the depressions, and then a polymer was immobilized on the chip substrate by means of amino-carboxyl condensation. The biomacromolecular layer, the small molecular layer, and part of the silicon dioxide film outside the truncated cone-shaped depressions were removed by polishing. The polished chip substrate was then cut and cleaned, and a cover plate with an untreated surface was used for encapsulation to give the biochip.

### Example 2

This example differs from Example 1 in that the depression is a cylindrical depression, as shown in FIG. 14; it can be considered that the depression includes a second geometric structure B that is symmetrical, where the bottom diameter of the second geometric structure B in a direction parallel to the surface of the chip is equal to its bottom diameter d in a horizontal direction on the side away from the surface of the chip, forming a shape that is consistent in width from the top to the bottom. For the cylindrical depression, the depth e in a direction perpendicular to the surface of the chip, the bottom diameter d in the horizontal direction on the side away from the surface of the chip, the included angle α between a side wall and the horizontal direction on the side away from the surface of the chip, and the distance f (i.e., center-to-center distance) between adjacent depressions are shown in Table 1. Table 1 shows the dimensional parameters of the depressions in the biochips provided in Examples 1-5.

### Example 3

This example differs from Example 1 in that the depression is a nearly conical depression, as shown in FIG. 12; it can be considered that the depression includes a first geometric structure A that is symmetrical, where the bottom diameter a of the first geometric structure A in a direction parallel to the surface of the chip is greater than its bottom diameter b in a horizontal direction on the side away from the surface of the chip, forming a shape that is wider at the top and narrower at the bottom. For the nearly conical depression, the depth c in a direction perpendicular to the surface of the chip, the bottom diameter a in the direction parallel to the surface of the chip, the bottom diameter b in the horizontal direction on the side away from the surface of the chip, the included angle α between a side wall and the horizontal direction on the side away from the surface of the chip, and the distance f (i.e., center-to-center distance) between adjacent depressions are shown in Table 1. Table 1 shows the dimensional parameters of the depressions in the biochips provided in Examples 1-5.

### Example 4

This example differs from Example 1 in that the biochip includes a silicon chip substrate, where depressions are formed in a honeycomb arrangement on the silicon chip substrate; that is, the depressions are arranged in rows and columns; the depressions of two adjacent rows are not located in the same column, and the depressions of alternate rows are located in the same column; the depressions of two adjacent columns are not located in the same row, and the depressions of alternate columns are located in the same row; the depression has a horn-shaped structure, as shown in FIG. 5. The depression includes, in sequence, a truncated cone-shaped depression A and a cylindrical depression B that are interconnected in a direction from a surface of the chip substrate toward a central part of the chip substrate; the truncated cone-shaped depression A is a symmetrical structure, with its bottom diameter a in a direction parallel to the surface of the chip being greater than its bottom diameter b in a horizontal direction on the side away from the surface of the chip, forming a shape that is wider at the top and narrower at the bottom; the cylindrical depression B is also a symmetrical structure, with its bottom diameter d being equal to its bottom diameter b, forming an overall shape that is consistent in width from the top to the bottom. The depth c in a direction perpendicular to the surface of the chip, the bottom diameter a in the direction parallel to the surface of the chip, and the bottom diameter b in the horizontal direction on the side away from the surface of the chip, and the included angle α between a side wall and the horizontal direction on the side away from the surface of the chip (for the truncated cone-shaped depression A), and the depth e in the direction perpendicular to the surface of the chip and the bottom diameter d in the horizontal direction on the side away from the surface of the chip (for the cylindrical depression B), as well as the distance f (i.e., center-to-center distance) between adjacent depressions, are shown in Table 1. Table 1 shows the dimensional parameters of the depressions in the biochips provided in Examples 1-5.

### Example 5

This example differs from Example 1 in that the depression is a flat-bottomed conical depression, as shown in FIG. 10; it can be considered that the depression includes a first geometric structure A that is symmetrical, where the bottom diameter a of the first geometric structure A in a direction parallel to the surface of the chip is greater than its bottom diameter b in a horizontal direction on the side away from the surface of the chip, forming a shape that is wider at the top and narrower at the bottom. For the flat-bottomed conical depression, the depth c in a direction perpendicular to the surface of the chip, the bottom diameter a in the direction parallel to the surface of the chip, the bottom diameter b in the horizontal direction on the side away from the surface of the chip, the included angle α between a side wall and the horizontal direction on the side away from the surface of the chip, and the distance f (i.e., center-to-center distance) between adjacent depressions are shown in Table 1. Table 1 shows the dimensional parameters of the depressions in the biochips provided in Examples 1-5.

**Table 1. Dimensional parameters of depressions in biochins provided in Examples 1-5**

| Example | a (nm) | b (nm) | c (nm) | d (nm) | e (nm) | f (nm) | α (°) |
|---|---|---|---|---|---|---|---|
| 1 | 250 | 400 | 500 | \ | 0 | 750 | 100 |
| 2 | \ | \ | 0 | 400 | 500 | 750 | 90 |
| 3 | 400 | 5 | 500 | \ | 0 | 750 | 60 |
| 4 | 400 | 250 | 200 | 250 | 300 | 750 | 20 |
| 5 | 400 | 250 | 500 | \ | 0 | 750 | 60 |

### Test Example 1

Amplification sequencing was performed on the biochips prepared in Examples 1-5 as follows:
Probes were introduced into the biochips provided in Examples 1-5 and immobilized in the microwells via the polymer. A nucleic acid library (a nucleic acid sample under test) was introduced and subjected to surface amplification to give an amplification cluster of the copies in the microwells.

Nucleic acid substrates ATCG containing a fluorophore and a polymerase were added for the base extension reaction. The influence signal of each base extension reaction was acquired, and the base type participating in each cycle of base extension reaction was determined through image analysis to determine the sequence of the nucleic acid library.

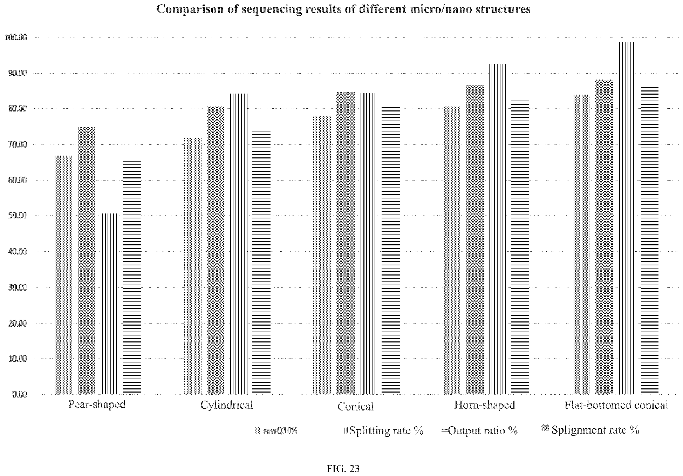

The light intensity of ATCG in the acquired fluorescent images of the chips provided in Examples 1-5 was summarized, and the results, as shown in FIG. 23, show that: when used for sequencing, the biochips with the micro/nano structures that are wider at the top and narrower at the bottom (Examples 3-5) exhibited a higher light intensity of ATCG in the fluorescence image and a higher copy number of the nucleic acid library in the depressions, as compared to the biochip with the micro/nano structure that is consistent in width from the top to the bottom (Example 2) or the biochip with the micro/nano structure that is narrower at the top and wider at the bottom (Example 1).

The summary of Q30, output ratio, splitting rate, and alignment rate during the amplification on the chips provided in Examples 1-5 are shown in FIG. 23.

The Q30 refers to the percentage of bases having a Q value of 30 or greater among all bases, and the Q value is calculated by the formula: Q = -10log₁₀P, where P is the error rate; thus, the Q30 may also be interpreted as: the percentage of bases with a base-calling error rate less than 0.1% in all sequenced bases; the Q30 reflects the sequencing quality, and a higher value indicates a better sequencing quality.

The output ratio refers to the proportion of valid reads, which are the reads that pass the filtering criteria, out of the total reads that generate signals and are recognized by the algorithm.

The splitting rate refers to the percentage of nucleic acid molecules in each labeled sample that can be aligned, relative to the total nucleic acid molecules in the mixed sample during mixed sequencing of multiple samples.

The alignment rate refers to the percentage of sequencing sequences that can be aligned to the standard sequence of the sample.

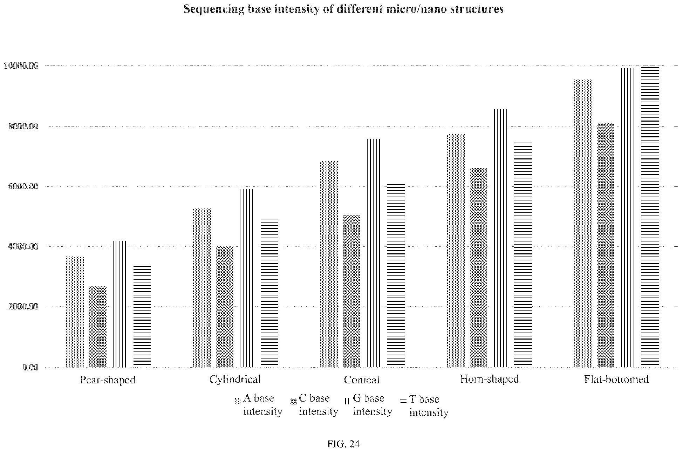

FIG. 24 shows that: when used for sequencing, the biochips with the micro/nano structures that are wider at the top and narrower at the bottom (Examples 3-5) achieved better sequencing results, as compared to the biochip with the micro/nano structure that is consistent in width from the top to the bottom (Example 2) or the biochip with the micro/nano structure that is narrower at the top and wider at the bottom (Example 1).

The above description is only illustrative of the preferred embodiments of the present disclosure, and it should be noted that those of ordinary skill in the art can make, without departing from the principle of the present disclosure, various improvements and modifications, and such modifications and modifications shall also be construed within the protection scope of the present disclosure.

## Claims

1. A chip, comprising a chip substrate, wherein the chip substrate is provided with regularly arranged depressions on at least one surface;
the depression comprises, in sequence, a first geometric structure and a second geometric structure that are interconnected in a direction from a surface of the chip substrate toward a central part of the chip substrate; the first geometric structure has a depth c of 0-100 µm in a direction perpendicular to the surface of the chip, a maximum dimension a of 10 nm-100 µm in a direction parallel to the surface of the chip, and a maximum dimension b of 10 nm-100 µm in a horizontal direction on the side away from the surface of the chip; an included angle α between a side wall of the first geometric structure and the horizontal direction on the side away from the surface of the chip is 10-170°;
the second geometric structure has a depth e of 0-100 µm in the direction perpendicular to the surface of the chip and a maximum dimension d of 0-100 µm in the horizontal direction on the side away from the surface of the chip; the depth c and the depth e are not simultaneously 0.

2. The chip according to claim 1, wherein the included angle α satisfies the following relationship: 10° ≤ α < 90°.

3. The chip according to claim 2, wherein the maximum dimension a is greater than the maximum dimension b, and the maximum dimension b is equal to the maximum dimension d;
optionally, the depth c is 50 nm-50 µm.

4. The chip according to claim 2, wherein the maximum dimension a is greater than the maximum dimension b, the maximum dimension b is greater than the maximum dimension d, and the maximum dimension d is not 0;
optionally, the depth c is 50 nm-50 µm;
optionally, the ratio of the depth c to the depth e is 1:1000-1000:1;

5. The chip according to claim 2, wherein the maximum dimension a is greater than the maximum dimension b, and the maximum dimension d is equal to 0;
optionally, the depth c is 50 nm-50 µm;
optionally, the depth e is 0.

6. The chip according to any one of claims 1-5, wherein the distance f between adjacent depressions is 10 nm-100 µm.

7. The chip according to any one of claims 1-6, wherein the surface is provided with at least one array unit having the regularly arranged depressions;
optionally, the array unit is one or more of a square array unit, a rectangular array unit, and a honeycomb array unit.

8. The chip according to any one of claims 1-7, wherein the chip comprises a polymer layer arranged on the surface provided with the depression;
optionally, the polymer layer is formed from a polymer containing a first functional group selected from one or more of sulfydryl, formyl, hydroxyl, carboxyl, amino, alkenyl, alkynyl, and azido;
optionally, the polymer layer is bonded to the surface of the chip substrate and/or the cover plate via a coupling molecule;
optionally, the coupling molecule has a molecular formula as follows: XₐSi(Y_{b})M_{c}Z, wherein
X is H, F, Cl, Br, I, C₁₋₆ alkyl, C₁₋₆ haloalkyl, or C₁₋₆ alkoxy;
Y is selected from C₁₋₁₀ alkyl;
M_{c} is selected from C₁₋₅₀ alkylene, -(OCH₂CH₂)_{c}-, and -(CH(OH)CH₂-)_{c};
Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl;
a, b, and c are integers meeting the following conditions: 0 ≤ a ≤ 3, 0 ≤ b ≤ 3, and a + b = 3; 1 ≤ c ≤ 50.

9. The chip according to any one of claims 1-8, wherein the chip further comprises a first film layer arranged between the chip substrate and/or the cover plate and the polymer layer, wherein the first film layer is made of a material different from that of the surface to which it is bonded.
optionally, the first film layer is selected from one or more of a metal film, a metal oxide film, and an inorganic oxide film;
optionally, the first film layer is made of at least one of Au, Al, and SiO₂;
optionally, the first film layer has a thickness of 10-1000 nm.

10. A method for preparing a chip, comprising the following step:
a) forming regularly arranged depressions on at least one surface of a substrate, wherein
the depression comprises, in sequence, a first geometric structure and a second geometric structure that are interconnected in a direction from a surface of a chip substrate toward a central part of the chip substrate; the first geometric structure has a depth c of 0-100 µm in a direction perpendicular to the surface of the chip, a maximum dimension a of 10 nm-100 µm in a direction parallel to the surface of the chip, and a maximum dimension b of 10 nm-100 µm in a horizontal direction on the side away from the surface of the chip; an included angle α between a side wall of the first geometric structure and the horizontal direction on the side away from the surface of the chip is 10-170°;
the second geometric structure has a depth e of 0-100 µm in the direction perpendicular to the surface of the chip and a maximum dimension d of 0-100 µm in the horizontal direction on the side away from the surface of the chip;
the depth c and the depth e are not simultaneously 0.

11. The method according to claim 10, wherein the substrate is the chip substrate and/or a cover plate.

12. The method according to claim 10, wherein the method further comprises:
b) forming a polymer layer on the depression;
optionally, b) comprises:
bonding a polymer containing a first functional group to the surface provided with the depression; and
polishing the polymer to remove the polymer outside the region where the depression is located, so as to form the polymer layer on the depression;
optionally, the polishing is chemical polishing and/or mechanical polishing.

13. The method according to any one of claims 8-12, wherein the polymer layer is bonded to the surface of the chip substrate and/or the cover plate via a coupling molecule;
optionally, the coupling molecule has a molecular formula as follows: XₐSi(Y_{b})M_{c}Z, wherein
X is H, F, Cl, Br, I, C₁₋₆ alkyl, C₁₋₆ haloalkyl, or C₁₋₆ alkoxy;
Y is selected from C₁₋₁₀ alkyl;
M_{c} is selected from C₁₋₅₀ alkylene, -(OCH₂CH₂)_{c}-, and -(CH(OH)CH₂-)_{c};
Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl;
a, b, and c are integers meeting the following conditions: 0 ≤ a ≤ 3, 0 ≤ b ≤ 3, and a + b = 3; 1 ≤ c ≤ 50.

14. The method according to any one of claims 8-13, wherein the method further comprises: before step a), or after step a) and before step b), preparing a first film layer on the surface provided with the depression, wherein the first film layer and the substrate are made of different materials;
optionally, the first film layer is selected from one or more of a metal film, a metal oxide film, and an inorganic oxide film;
optionally, the first film layer is made of at least one of Au, Al, and SiO₂;
optionally, the first film layer has a thickness of 10-1000 nm.

15. The method according to any one of claims 8-14, wherein the substrate comprises at least the chip substrate, and the method further comprises:
bonding the cover plate to the surface of the chip substrate provided with the depression using an adhesive material, and forming a channel in the adhesive material in a length direction of the chip.
